(19) Europäisches Patentamt
European Patent Office
Office européen des brevets

(11) Publication number: 0 081 893
B1

(12) **EUROPEAN PATENT SPECIFICATION**

(45) Date of publication of patent specification: **04.03.87**

(21) Application number: **82201609.3**

(22) Date of filing: **15.12.82**

(51) Int. Cl.$^4$: **C 07 D 493/08,** C 07 D 493/18, C 07 F 9/38, C 07 F 9/09, A 01 N 43/90, A 01 N 57/16, A 01 N 57/24 // (C07D493/08, 307:00, 307:00),(C07D493/18, 307:00, 307:00, 303:00),(C07D493/08, 311:00, 307:00),(C07D493/08, 311:00, 311:00)

(54) **Oxabicycloalkane herbicides.**

(30) Priority: **16.12.81 US 331094**
**13.09.82 US 416572**

(43) Date of publication of application:
**22.06.83 Bulletin 83/25**

(45) Publication of the grant of the patent:
**04.03.87 Bulletin 87/10**

(84) Designated Contracting States:
**AT BE CH DE FR GB IT LI LU NL SE**

(56) References cited:
**EP-A-0 000 002**
**DE-A-2 724 677**
**FR-A-2 370 741**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Payne, George Bernson**
**308 Harrow Court**
**Modesto, CA 95350 (US)**
Inventor: **Soloway, Samuel Barney**
**3401 Mansfield Lane**
**Modesto, CA 95350 (US)**
Inventor: **Powell, James Edward**
**16093 East Eugenia**
**Ripon, CA 95366 (US)**
Inventor: **Roman, Steven Alan**
**28902 Walker Lane**
**Fulshear, TX 77441 (US)**
Inventor: **Kollmeyer, Willy Dietrich**
**1008 Stratford Lane**
**Modesto, CA 95350 (US)**

(74) Representative: **Hunter, Keith Roger Ian et al**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 081 893 B1

**Description**

This invention relates to novel oxabicycloalkane derivatives useful as herbicides.

European Patent Application No. 0000002, and German Offenlegungschrift No. 2724677, disclose that certain tetrahydrofuran derivatives are useful as herbicides. It has now been found that certain novel bicyclic compounds containing an oxygen atom bridging the rings have useful herbicidal properties.

The present invention provides a compound of the general formula

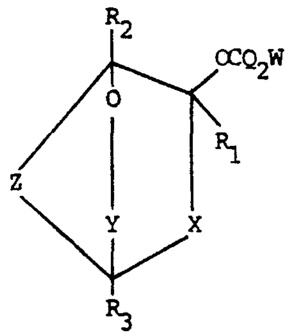

(I)

wherein

X is $—CR_4R_4—$;

Y is $(—CR_5R_6—)_n$ in which n is 0 or 1;

Z is $(CR_7R_7—)_p$ in which p is 1, 2 or 3;

$R_1$ is a hydrogen atom or $C_{1-6}$ alkyl group optionally substituted by up to 3 fluorine, chlorine and/or bromine atoms;

$R_2$ is a hydrogen atom or a $C_{1-6}$ straight-chain alkyl group;

$R_3$ is a hydrogen atom; a cyano group; a $C_{1-10}$ alkyl group; a $C_{1-6}$ alkyl group which is substituted by one or more halogen atoms or by a hydroxy group, a cyano group, a $C_{1-6}$ alkoxy group, a phenoxy group, a $C_{1-6}$ alkylsulphonyl group, a phenylsulphonyl group, a benzylsulphonyl group, an azido group, a $C_{1-6}$ alkoxycarbonyl group, a benzyloxycarbonyl group, a hydroxycarbonyl group, a phosphoryl group, a phosphoryloxy group, or an amine oxide, carbamoyl or thiocarbamoyl group in which each nitrogen is optionally substituted by 1 or 2 $C_{1-4}$ alkyl groups; a $C_{2-4}$ alkenyl or alkynyl group; an aryl or aralkyl group, each containing from 6 to 11 carbon atoms including 1 to 4 carbon atoms in any alkyl portion, optionally ring substituted by one or more fluorine, chlorine and/or bromine atoms or by a $C_{1-2}$ alkyl or alkoxy group, each optionally substituted by one or more fluorine and/or chlorine atoms; a group $—CSNH_2$; a group $—CO_2R_8$ or $—CON(R_8)_2$ in which $R_8$ is a hydrogen atom or a $C_{1-6}$ alkyl group; or an acetyl group or an oxime or acetal derivative of said group;

each $R_4$ is independently a hydrogen atom; a $C_{1-6}$ alkyl group optionally substituted by up to 3 halogen atoms; a hydroxy group; or a $C_{1-4}$ alkoxy group; or one of $R_4$ and $R_1$ together form a carbon-carbon bond;

$R_5$ and $R_6$ each independently is a hydrogen atom or a $C_{1-2}$ alkyl group; or when located on a carbon atom adjacent to the ring oxygen atom then $R_5$ and $R_6$ may together form an alkylene group containing 4 or 5 carbon atoms;

each $R_7$ independently is a hydrogen atom or a $C_{1-4}$ alkyl group optionally substituted by up to 3 halogen atoms; or when n is 0 then $R_7$ may also be a chlorine or bromine atom, or two of $R_7$ when located on adjacent carbon atoms together form an epoxide ring or a carbon-carbon bond; or when n is 1, then one $R_7$ on the carbon adjacent to the carbon bearing $R_2$ is a hydroxy group, a $C_{7-11}$ aralkoxy group, or a $C_{1-4}$ alkoxy group, and the other $R_7$ is a hydrogen atom;

both of Q are hydrogen atoms or fluorine atoms; and

W represents a cyano group; a $C_{2-4}$ alkenyl or alkynyl group; a pyrimidinyl, pyrazinyl, pyridazinyl, pyridyl, furyl, naphthyl, imidazolyl, triazolyl, thiadiazolyl, 2-quinolinyl, 1-isoquinolinyl, pyrrolyl, N-methylimidazolyl, N-methylpyrazolyl, isoxazolyl, oxazolyl, isothiazolyl, thiazolyl, thienyl or 5-methyl-2-furyl group; or a cyclohexenyl group, or a cycloalkyl group having up to 6 carbon atoms optionally substituted by a $C_{1-3}$ alkyl group; or a $C_{3-10}$ secondary alkyl group; or a phenyl group which is unsubstituted or substituted by one or more of the following moieties: hydroxy, cyano, nitro, halogen, $C_{1-3}$ alkoxy, haloalkoxy, alkylthio, haloalkylthio, alkylsulphinyl, haloalkylsulphinyl or alkylsulphonyl; alkenyl or alkynyl of up to 4 carbon atoms; phenyl, phenoxy, benzyl or benzyloxy; amino, aminocarbonyl and carboxyl in each of which hydrogen may be replaced by $C_{1-4}$ alkyl; and $C_{1-3}$ alkyl which is itself optionally substituted by halogen, hydroxy, $C_{1-4}$ alkylthio, $C_{1-4}$ alkoxy and amino.

Unless otherwise stated, throughout this Specification and claims, an aliphatic group preferably has up to 6, especially up to 4, carbon atoms, and an aryl group is preferably a phenyl group. Preferred halogen atoms are fluorine, chlorine and bromine atoms.

Preferably, W is a cyano group; a $C_{2-4}$ alkenyl or alkynyl group; a pyrimidinyl group; a pyrazinyl group; a pyridazinyl group; a pyridyl group; a furyl group; a naphthyl group; or a phenyl group optionally substituted by one or more, especially 1 to 3, of hydroxy; cyano; halogen; $C_{1-3}$ alkoxy, alkylthio or

alkylsulphinyl, each optionally substituted by halogen; benzyloxy; amino, carboxyl or aminocarbonyl; or $C_{1-3}$ alkyl optionally substituted by halogen, hydroxy, alkoxy, alkylthio or amino, optionaly substituted by ($C_{1-4}$ alkyl)carbonyl.

It is further preferred that W is a $C_{2-4}$ alkenyl or alkynyl group; a 4-pyrimidinyl group; a 2-pyrazinyl group; a 3-pyridazinyl group; a 2-pyridyl group; a 2-furyl group; or a phenyl group optionally substituted by one or more of halogen, cyano, amino, $C_{1-3}$ alkoxy or alkylthio each optionally substituted by one or more fluorine and/or chlorine atoms, or $C_{1-2}$ alkyl optionally substituted by one or more fluorine and/or chlorine atoms, or by hydroxy, $C_{1-2}$ alkoxy or $C_{1-2}$ alkylthio.

Most preferably W is a 2-pyridyl group, or a phenyl group which is unsubstituted or, especially, substituted by one or two substituents selected from methyl groups, chlorine atoms and fluorine atoms. Such substituents are preferably in the 2- or 2,6-positions. Typical groups W include 2-pyridyl, phenyl, 4-fluorophenyl, 2-chlorophenyl, 2-fluorophenyl, 2-methylphenyl or 2,6-dichlorophenyl. Especially in compounds in which n is 0 or 1, and p is 2, W may also advantageously represent an ethynyl group.

Each $R_4$ preferably independently represents a hydrogen atom or a methyl or ethyl group.

If n is 1, most preferably each of $R_5$ and $R_6$ independently represents a hydrogen atom or a methyl or ethyl group. If located on a carbon atom adjacent to the ring oxygen atom, each of $R_5$ and $R_6$ is preferably a methyl or ethyl group, otherwise each of $R_5$ and $R_6$ is preferably a hydrogen atom. Preferably n is 0 or 1.

Preferably each $R_7$ independently represents a hydrogen atom or an alkyl group, especially a methyl or ethyl group, or when n is 0, a chlorine or bromine atom or, when n is 1, one $R_7$ on the carbon atom adjacent to the carbon atom bearing $R_2$ is a hydroxy, methoxy, ethoxy or benzyloxy group, the or each other $R_7$ being a hydrogen atom. Most preferably each $R_7$ represents a hydrogen atom. Preferably p is 2.

Preferably the sum of n and p is 2 or 3.

$R_1$ is preferably a methyl group or, especially, a hydrogen atom.

$R_2$ is preferably a hydrogen atom or a straight-chain alkyl group having 1 to 3 carbon atoms. Most preferably $R_2$ is a methyl or ethyl group.

$R_3$ is preferably a hydrogen atom; a $C_{1-6}$ alkyl group optionally substituted by up to 3 flourine, chlorine and/or bromine atoms or by a hydroxy, cyano, $C_{1-6}$ alkoxy, $C_{1-6}$ alkylsulphonyl, phenylsulphonyl or benzylsulphonyl group; a $C_{2-4}$ alkenyl or alkynyl group; or an aryl or aralkyl group containing 6 to 11 carbon atoms and 1 or 2 carbon atoms in any alkyl portion, optionally ring substituted by one or more fluorine, chlorine and/or bromine atoms or by a $C_{1-2}$ alkyl or alkoxy group optionally substituted by one or more fluorine and/or chlorine atoms.

Most preferably $R_3$ represents a hydrogen atom or a $C_{1-3}$ alkyl group optionally substituted by one or more halogen atoms. Especially preferred groups $R_3$ are methyl, ethyl, isopropyl and 1-chloro-1-methylethyl groups. When n is 0, $R_3$ is preferably an isopropyl group.

Preferably each Q is a hydrogen atom.

Typical compounds of the general formula I include those in which, for example, $R_3$ is a hydrogen atom or a $C_{1-10}$ alkyl group or when n is 0, then $R_3$ may additionally be a cyano group, a $C_{1-6}$ alkyl group substituted by a hydroxy group, a cyano group, a $C_{1-6}$ alkoxy group, an aryloxy group, a $C_{1-6}$ alkylsulphonyl group, a phenylsulphonyl group, a benzylsulphonyl group, an azido group, a ($C_{1-6}$ alkoxy)carbonyl group, a hydroxycarbonyl group, a phosphoryl group, a phosphoryloxy group, or an amine oxide, carbamoyl or thiocarbamoyl group in which each nitrogen is substituted by hydrogen or by 1 or 2 $C_{1-4}$ alkyl groups; or $R_3$ is an alkenyl or alkynyl group containing 2 to 4 carbon atoms; an aryl or aralkyl group, each containing from 6 to 11 carbon atoms including 1 to 4 carbon atoms in any alkyl portion and optionally ring substituted by one or more substituents selected from fluorine, chlorine and/or bromine atoms, or by a $C_{1-2}$ alkyl or alkoxy group, each optionally substituted by one or more fluorine and/or chlorine atoms; or $R_3$ is a group $—CO_2R_8$ or $—CON(R_8)_2$ in which $R_8$ is a hydrogen atom or a $C_{1-6}$ alkyl group;

each $R_4$ is independently a hydrogen atom or a $C_{1-6}$ alkyl group optionally substituted by up to 3 halogen atoms; or one of $R_4$ and $R_1$ together form a carbon-carbon bond; and

W is an alkenyl or alkynyl group containing from 2 to 4 carbon atoms; a 4-pyrimidyl group; a 2-pyrazinyl group; a 3-pyridazinyl group; a 2-pyridyl group; a 2-furyl group; or a phenyl group optionally substituted by one or more of halogen, cyano, amino, $C_{1-3}$ alkoxy or alkylthio, each optionally substituted by one or more fluorine and/or chlorine atoms, or $C_{1-2}$ alkyl optionally substituted by one or more fluorine and/or chlorine atoms, hydroxy, $C_{1-2}$ alkoxy, or $C_{1-2}$ alkylthio.

The compounds of the general formula I exhibit geometric and optical isomerism. The invention includes all individual isomers and mixtures thereof. Often, different isomers will exhibit different levels of herbicidal activity. Thus mixtures containing greater than 70%, preferably greater than 80% and most preferably greater than 95% of a desired isomer or mixture of isomers may be useful.

Most preferred compounds according to the invention are those in which n is 0 and p is 2, or n is 1 and p is 2. These compounds have the general formulae:

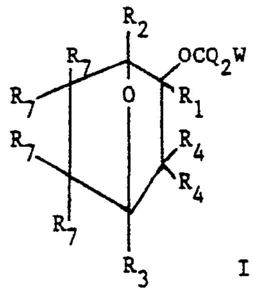

I (a)

and

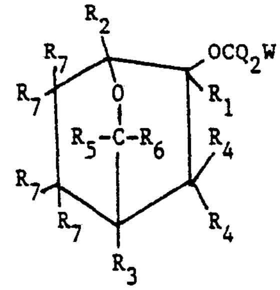

I(b)

in which the various symbols have the meanings and preferred meanings given for the general formula I. Especially preferred compounds I(a) are those in which $R_2$ is a methyl group and $R_3$ is an isopropyl or 1-chloro-1-methylethyl group, or $R_2$ and $R_3$ are both ethyl groups. Most preferably all of $R_1$, $R_4$ and $R_7$ represent hydrogen atoms. In general, compounds of the formula I(a) in which the $OCQ_2W$ group is *exo* tend to have a greater herbicidal activity than the corresponding *endo* compounds.

Especially preferred compounds of formula I(b) are those in which $R_2$ represents a methyl group, and all of $R_1$, $R_4$ and $R_7$ are hydrogen atoms. Compounds of the formula I(b) in which the $OCQ_2W$ group is in an *endo* configuration tend to have a greater herbicidal activity than the corresponding *exo* compounds.

The invention also provides a process for the preparation of a compound of the general formula I, which comprises reacting a compound of the general formula

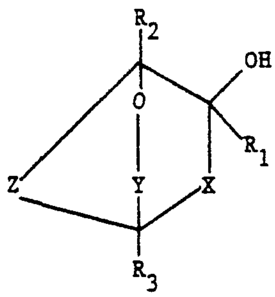

(II)

in which $R_1$, $R_2$, $R_3$, X, Y and Z have the meanings given for the general formula I, with a compound of the general formula $WCQ_2L$, in which W and Q have the meanings given for the general formula I, and L represents a suitable leaving group.

L may for example represent a halogen atom, especially a bromine, chlorine or iodine atom, or an organic sulphonyloxy group, for example a mesyloxy or tosyloxy group.

The reaction is preferably carried out in the presence of a strong base, for example an alkali metal hydride, hydroxide or carbonate, for example sodium hydride, sodium hydroxide or potassium carbonate. Preferably an inert solvent is used; typical solvents include ethers, sulphoxides, aromatic hydrocarbons and chlorinated hydrocarbons, for example diethyl ether, tetrahydrofuran, dimethyl sulphoxide, toluene and methylene chloride. It may be desired to include a catalyst for the reaction; suitable catalysts include organic bases, such as tertiary amines and quaternary ammonium compounds, for example triethylamine or tetrabutylammonium iodide. Suitable temperatures for the reaction are, for example from 0° to 120°C, preferably from 20° to 100°C.

The compounds of the general formula II may be obtained generally by one or more of the following routes: directly by (a) epoxidation-cyclization of unsaturated cyclic alcohols, with or without isolation of epoxy alcohol intermediates; and indirectly by (b) photochemical ring closure of unsaturated ketones; (c) ring contraction of diazo ketones; (d) Diels-Alder reactions of furans with dienophiles.

In (b), the photochemical ring closure may be accomplished by application of conventional techniques on unsaturated ketones, such as described in Furth, et al., *Tetrahedron Letters,* No. 48, pages 4259-62 (1975) for unsaturated ketones.

In (d), the Diels-Alder type adducts of furans with dienophiles may require vigorous reaction conditions, including high pressure and low temperature, for example, as described in Dauben, W. G. et al., J. *Amer. Chem. Soc., 102* page 6894 (1980). When the dienophile is nitroethylene, the resulting product may be hydrogenated, then oxidized to the ketone and reduced to the corresponding alcohol, e.g. by treatment with a hydride or metal. When this alcohol has a specific isomeric form, e.g. the *endo* form, it can be epimerized with base or aluminium isopropoxide in the presence of a ketone to produce the other corresponding *exo* alcohol.

A preferred method, however, for the preparation of a compound of the general formula II is the direct epoxidation — cyclisation (a) above, of the corresponding unsaturated cyclic alcohol. This method is

described in detail below, using compounds in which m is 1, n is 0 and p is 2 as an example. In this case, the starting material is a cyclohex-3-en-1-ol.

The epoxidation of a cyclohex-3-en-1-ol into the corresponding *cis*-epoxy-alcohol may be effected by action of an oxidizing agent, particularly a peroxide, such as m-chloroperbenzoic acid, peracetic acid, tert-butyl hydroperoxide (TBHP) or equivalent peroxide reagents. Preferably, an oxidation with TBHP is conducted in the presence of an appropriate transition metal catalyst. Suitable transition metal catalysts are complexes of metals of atomic numbers 22—31, 40—49 and 72—81. Preferably, the complex is an organic complex, for example, with beta-diketones, o-hydroxybenzaldehydes or o-hydroxybenzophenones and particularly with acetylacetone. While any of these transition metal catalysts can be used, those of vanadium or molybdenum are preferred; for example, vanadium (IV) bis(2,4-pentanedionate) oxide is preferred. The reaction is suitably conducted in the presence of an inert solvent such as a chlorinated hydrocarbon, ether or hydrocarbon. The reaction is conveniently conducted at a temperature in the range of from about −10°C to about 50°C or slightly above. Generally, the temperature is from about −5°C to about 40°C, preferably from about 10°C to about 30°C. The molar ratio of reactants can vary. Generally, a suitable molar ratio of cyclohex-3-en-ol to oxidizing agent is from about 0.8 to about 1.0. The resulting product epoxy-alcohol may be purified or converted without isolation into a 2-*exo*-hydroxy-7-oxabicyclo[2,2,1]heptane by cyclization as described below.

The cyclization step is carried out by treating the epoxy-alcohol with an acid, and, surprisingly, gives a relatively high yield of produce having the *exo*-hydroxy configuration in the resulting 7-oxabicyclo[2,2,1]heptan-2-ol. Many acids will catalyze this reaction, but a relatively strong acid such as sulphuric or a sulphonic acid is preferred. Preferably, the acid is methanesulphonic acid, benzenesulphonic acid or, especially, p-toluenesulphonic acid. The reaction is suitably carried out in a solvent of the type previously described for use in the preparation of the epoxy-alcohol. The reaction may be conveniently conducted at a temperature in the range of from about 0°C to about 50°C or slightly above. Generally, the temperature is from about 5°C to about 40°C, preferably from about 10°C to about 30°C. The molar ratio of reactants can vary. Generally, the preferred molar ratio of acid to epoxy-alcohol is from 0.01 to about 0.10, especially from 0.02 to 0.04.

In situations where the *endo* form is desired, it can be obtained by oxidation of the 2-*exo*-hydroxy compound to the corresponding ketone followed by reduction of the ketone with sodium borohydride.

The 3-cyclohexen-1-ols used as starting materials can be synthesized as described below or obtained from natural sources (which offer the advantage of optically-active materials).

(a) where $R_2$ is methyl and $R_3$ is isopropyl and the remaining R's are hydrogen, the compound is terpinen-4-ol, which occurs naturally, or can be prepared by epoxidation of terpinolene, followed by reduction of the epoxide.

(b) Substituted-1-oxaspiro(2,5)oct-5-enes are useful for preparing 3-cyclohexen-1-ols where $R_3$ is substituted by groups of the type OH, OR, SR, $NR_2$, Cl, $N_3$, $P(O)(OR)_2$. Typical reactions are included in the Examples herein. Compounds where $R_3$ is alkenyl may be made by rearrangement of the spiro compound by treatment with protic or Lewis acids. Where $R_3$ is substituted by an amine oxide group, a spiro compound may be treated with the appropriate dialkylamine in the presence of a catalyst such as triethylaluminium; the subsequent epoxidation step produces the amine oxide.

(c) Preparation of 3-cyclohexen-1-ols can be effected from *p* substituted phenols by procedures of the literature for the Birch-type reduction of derivatives of benzene, many of which are detailed in Rodd's Chemistry of Carbon Compounds, Second Edition, *Vol. II, Part B,* pages 1—4 (1968).

(d) Where $R_3$ is substituted by CN, a 4-substituted-3-cyclohexen-1-one may be treated with an alpha-bromoalkanenitrile in the presence of zinc dust.

(e) Where $R_3$ is $—CO_2R_8$, $—CON(R_8)_2$, —CN, $—CSNH_2$, or alkyl, the 3-cyclohexen-1-ols can be prepared starting from suitable Diels-Alder adducts. For example, methyl pyruvate may be converted by known procedures to its enol acetate and treated with isoprene to produce a Diels-Alder adduct. Hydrolysis of the acetate function affords 1-hydroxy-4-methyl-3-cyclohexene-1-carboxylic acid methyl ester. Appropriate modification by known methods of functional groups $R_3$ may be carried out.

Compounds of the general formula II in which m is 1, n is 1 and p is 2 are also most advantageously prepared by epoxidation-cyclisation, starting from cyclohex-3-en-1-methanols by the methods described above. The starting unsaturated alcohols may for example be prepared by the following methods.

(a) From natural sources, e.g. α-terpineol or α-pinene.

(b) By Diels-Alder reactions using readily available dienophiles, for example acrylate esters, acrolein, methacrolein, methyl vinyl ketone, allyl alcohol and crotonate esters, and dienes for example isoprene or 2,3-dimethylbutadiene.

Details of such reactions are given in Rodd's Chemistry of Carbon Compounds, 2nd Edition, Vol. II, Part B, pages 5—6 (1968), and in the Examples herein.

The compounds according to the invention exhibit useful herbicidal properties. The invention therefore also provides a herbicidal composition which comprises a compound of the general formula I together with a carrier and/or a surface-active agent. The invention also provides a process for the control of unwanted plants at a locus, which comprises treating the locus with a compound or a composition according to the invention. The locus may for example be a crop area, for example a crop area containing plants or seeds of cotton, soybeans, peanuts, wheat or rice. The dosage used is preferably in the range of

from 0.05 to 10, especially 0.1 to 5, kilograms of active material per hectare. A composition according to the invention preferably contains from 0.5 to 95% by weight of active material.

By "carrier" is meant a solid or fluid material, which may be inorganic or organic and of synthetic or natural origin, with which the compound of the invention is mixed or formulated to facilitate its application to the plant, seed, soil or other object to be treated, or its storage, transport or handling.

Suitable solid carriers are natural and synthetic clays and silicates, for example, natural silicas such as diatomaceous earths; magnesium silicates, for example, talcs, magnesium aluminium silicates, for example, attapulgites and vermiculites; aluminium silicates, for example, kaolinites, montmorillonites and micas; calcium carbonates; calcium sulphate; synthetic hydrated silicon oxides and synthetic calcium or aluminium silicates; elements, for example, carbon and sulphur; natural and synthetic resins for example, coumarone resins, polyvinyl chloride and styrene polymers and copolymers; solid polychlorophenols; butumen; waxes, for example, beeswax, paraffin wax, and chlorinated mineral waxes; and solid fertilizers, for example, superphosphates.

Examples of suitable fluid carriers are water; alcohols, for example, isopropanol; glycols; ketones, for example, acetone, methyl ethyl ketone, methyl isobutyl ketone and cyclohexanone; ether, for example, tetrahydrofuran; aromatic hydrocarbons, for example, benzene, toluene and xylene; petroleum fractions, for example, kerosene or light mineral oils; chlorinated hydrocarbons, for example, carbon tetrachloride, perchloroethylene, trichloroethane and chlorobenzene. Liquified normally vaporous compounds may be used. Mixtures of different liquids are often suitable.

A surface-agent may be an emulsifying agent or a wetting agent; it may be nonionic or ionic. Any of the surface-active agents usually applied in formulating herbicides or insecticides may be used. Examples of suitable surface-active agents are the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohols and alkylphenols, for example, p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example, sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxides.

The compositions may for example be formulated as wettable powders, dusts, granules, solutions, emulsifiable concentrates, emulsions, suspension concentrates and aerosols. Wettable powders are usually compounded to contain 25, 50 and 75% by weight of toxicant and usually contain in addition to solid carrier, 3—10% by weight of a dispersing agent, 15% of a surface-active agent and where necessary, 0—10% by weight of stabilizer(s) and/or other additives such as penetrants or stickers. Dusts are usually formulated as a dust concentrate having a similar composition to that of a wettable powder but without a dispersant or surface-active agent, and are diluted in the field with further solid carrier to give a composition usually containing 0.5—10% by weight of toxicant. Granules are usually prepared to have a size between 10 and 100 BS mesh (1.676—0152 mm), and may be manufactured by agglomeration or impregnation techniques. Generally, granules will contain 0.5—25% by weight toxicant and 0—1% by weight additives such as stabilizers, slow-release modifiers and binding agents. Emulsifiable concentrates usually contain, in addition to the solvent and, when necessary, cosolvent, 10—50% weight per volume toxicant, 2—20% weight per volume emulsifiers and 0—20% weight per volume of appropriate additives such as stabilizers, penetrants and corrosion inhibitors. Suspension concentrates are compounded so as to obtain a stable, non-sedimenting, flowable product and usually contain 10—75% weight toxicant, 0.5—5% weight of dispersing agents, 1—5% of surface-active agent, 0.1—10% weight of suspending agents, such as defoamers, corrosion inhibitors, stabilizers, penetrants and stickers, and as carrier, water or an organic liquid in which the toxicant is substantially insoluble; certain organic solids or inorganic salts may be dissolved in the carrier to assist in preventing sedimentation or an antifreeze agent for water.

Aqueous dispersions and emulsions, for example, compositions obtained by diluting a wettable powder or a concentrate with water, also are suitable. The said emulsions may be of the water-in-oil or of the oil-in-water type, and may have a thick mayonnaise-like consistency.

The compositions may also contain other ingredients, for example, other compounds possessing pesticidal, especially insecticidal, acaricidal, herbicidal or fungicidal properties.

The invention is illustrated by the following examples. The identities of the products, including intermediates, were confirmed by elemental, infra red and nuclear magnetic resonance (NMR) analyses as necessary.

Examples 1 to 12

These examples illustrate the preparation of various unsaturated cyclic alcohols useful as intermediates in the preparation of compounds of the general formula I. It should be noted that the preparation of certain of intermediates for the preparation of the compounds of the general formula I is the subject of the co-pending application published as EP—A—0081892.

### Example 1

1,4-Diethyl-3-cyclohexen-1-ol

To a stirred refluxing mixture of 600 ml. of dry diethyl ether and 1600 ml of liquid ammonia was added 136 g of p-ethylanisole. After 15 minutes, there was added portionwise, at −35 to −32°C, 26.4 g of lithium ribbon over 0.5 hour. After an additional 15 minutes, 193 g of dry ethanol was added dropwise at −35 to −32°C. Stirring was continued until the blue colour disappeared, and the ammonia was allowed to evaporate on standing overnight. The residue was poured into 1 l of ice water and extracted twice with diethyl ether. The combined ether extracts concentrated to a volume of about 300 ml were stirred with 250 ml of water containing 46 g of oxalic acid overnight at ambient temperature. This mixture was diluted with 1 liter of water and extracted twice with diethyl ether. The combined ether extracts were washed with 5% sodium bicarbonate and then with water. After drying, the ether solution was vacuum-concentrated to a residue of 104.4 g of 4-ethyl-3-cyclohexen-1-one; it was 94% pure. A solution of 10.0 g of this product in 25 ml diethyl ether was added dropwise to a stirred solution of 35 ml of 3.2 M ethereal ethyl magnesium bromide in 75 ml of dry diethyl ether at gentle reflux. After one hour longer at reflux, the mixture was cooled and treated dropwise with 80 ml of water. The aqueous layer was extracted with diethyl ether and the combined ether layers were dried, concentrated, and Claisen-distilled to give 7.3 g of the desired product, b.p. 82—86°C (5 mm Hg; 667 Pa).

### Example 2

4-Methyl-1-(1-methyl-1-(phenylthio)ethyl)-3-cyclohexen-1-ol

To a stirred solution of 76 g of 2,2,6-trimethyl-1-oxaspiro-(2,5)oct-5-ene in 300 ml of n-pentanol were added 2.0 g of 60% sodium hydride and 60 g of thiophenol. After 18 hours reflux, the mixture was vacuum concentrated at 90—95°C. The residue was dissolved in methylene chloride and washed twice with 2N sodium hydroxide. The dried solution was Claisen-distilled to give 106 g of crude product, b.p. 120—125°C (0.1 mm Hg; 13 Pa). Recrystallization from 250 ml of hexane gave 69.1 g of the desired product, m.p. 73—74°C.

### Example 3

(±)-4-Methyl-1-(1-methyl-1-(methylthio)ethyl)-3-cyclohexen-1-ol

To a stirred solution of 15.2 g of 2,2,6-trimethyl-1-oxaspiro(2,5)oct-5-ene in 100 ml of N,N-dimethylacetamide was added 4.4 g of 60% sodium hydride. The mixture was cooled to 5—10°C and saturated with methyl mercaptan. Stirring was continued while heating the reaction mixture to 100°C over a two and one-half hour period. After an additional hour at 100°C, the reaction mixture was poured into water and extracted twice with n-pentane. The combined extracts were washed with water, dried and Claisen-distilled to give 15.8 g of the desired product, b.p. 83—85°C (0.2 mm Hg; 27 Pa).

### Example 4

(±)-4-Methyl-1-(1-chloro-1-methylethyl)-3-cyclohexen-1-ol

To a stirred solution of 15.2 g of 2,2,6-trimethyl-1-oxaspiro-(2,5)oct-5-ene in 200 ml of diethyl ether held at −10°C was added dropwise 32 ml of 3.8 N ethereal hydrogen chloride. After one hour at 0—5°C, the mixture was washed with three 50 ml portions of water, dried and distilled to give 14.5 g of the desired product, b.p. 70—75°C (0.4 mm Hg; 53 Pa).

### Example 5

(±)-1-Hydroxy-alpha, alpha,4-trimethyl-3-cyclohexene-1-acetonitrile

To a stirred mixture of 7.0 g of zinc dust 0.45 g of mercuric chloride and 4 ml of terahydrofuran was added dropwise over 45 minutes at 20—25°C a mixture of 9.7 g of 4-methyl-3-cyclohexen-1-one, 13.7 g of alpha-bromoisobutyronitrile and 25 ml of tetrahydrofuran. After an additional hour at 25°C, the reaction mixture was cooled to 5—10°C and treated dropwise with 50 ml of cold 10% sulphuric acid. To this was added 100 ml of methylene chloride, and the mixture was filtered. The filtrate was diluted with 100 ml of water and extracted twice with 100 ml portions of methylene chloride. The combined methylene chloride extracts were washed with bicarbonate, dried and Claisen-distilled to give 11.3 g of the desired product, b.p. 92—102°C (0.25 mm Hg; 33 Pa).

### Example 6

(±)-1-(1-hydroxy-1-methylethyl)-4-methyl-3-cyclohexen-1-ol

A mixture of 26.0 g of 2,2,6-trimethyl-1-oxaspiro(2,5)oct-5-ene and 250 ml of 1% sulphuric acid was stirred magnetically for 20 hours, then extracted with four 100 ml portions of methylene chloride. The combined methylene chloride extracts were washed, dried, concentrated and Claisen-distilled to give 22.4 g of the desired product, b.p. 78—81°C (0.15 mm Hg; 20 Pa).

### Example 7

(±)-4-Methyl-1-(1-methoxy-1-methylethyl)-3-cyclohexen-1-ol

To a stirred solution of 0.8 g of *p*-toluenesulphonic acid in 125 ml of methanol held at 3—5°C was added dropwise over 0.5 hour a solution of 15.2 g of 2,26-trimethyl-1-oxaspiro(2,5)oct-5-ene in 25 ml of methanol.

After an additional 2 hours at 5°C and 2 hours at 5—20°C, the mixture was treated with 2 ml of 15% sodium hydroxide and concentrated at a water pump at below 60°C. The residue was dissolved in methylene chloride, washed, dried and Claisen-distilled to give 15.6 g of the desired product, b.p. 70°C (0.2 mm Hg; 27 Pa).

Example 8

1-(4-Methyl-3-cyclohexen-1-yl)cyclopentan-1-ol

To a stirred suspension of 46.8 g of magnesium in 250 ml of tetrahydrofuran was added 182 g of 1,4-dibromobutane in 700 ml of dry tetrahydrofuran over 1 hour. The reaction mixture was maintained at reflux for 2 more hours and then cooled to room temperature. A solution of 100 g of the methyl ester of 4-methyl-3-cyclohexene-1-carboxylic acid in 20 ml of dry tetrahydrofuran was added at 20—30°C with cooling by an ice bath. The reaction mixture was stirred at room temperature for 2 days, and the excess Grignard reagent was decomposed by careful addition of dilute sulphuric acid. Most of the tetrahydrofuran was evaporated, and the reaction product was extracted with diethyl ether. The ether phase was washed successively with water and saturated NaCl solution, dried with $MgSO_4$, and stripped to give 120 g of crude product as an oil. Multiple redistillation of the oil gave 32 g of product b.p. 90—92°C (0.5 mm Hg; 67 Pa).

Example 9

(±)-alpha-Terpineol

64.5 g of dichloroacetic acid was added dropwise over 20 minutes at 5—6°C to a magnetically stirred mixture of 68.0 g of (+)-alpha-pinene ($[alpha]_D$ + 47.1°) and 9.0 g of water. After stirring overnight at 5—30°C the mixture was extracted with 400 ml of methylene chloride. The extract was washed successively with water, aqueous potassium carbonate solution and water, dried and Claisen distilled at 4 mm Hg (533 Pa) to give 47.5 g of the desired (+)-alpha-terpineol, b.p. 71—83°C. Redistillation through a micro Vigreaux column gave 40.5 g of (+)-alpha-terpineol with $[alpha]_D$ + 79.3° ($CHCl_3$), b.p. 58—60°C (1 mm Hg; 133 Pa).

Example 10

Alpha, alpha, 3-trimethyl-3-cyclopentene-1-methanol

Ethyl acetoacetate (125.8 g) in 400 ml of toluene was added dropwise to a suspension of sodium hydride (68.3 g of a 60% oil dispersion) in 1 L of toluene under an $N_2$ blanket at −6°C to 2°C. After 20 minutes, 1,4-dibromo-2-methyl-2-butene (220.6 g) in 300 ml of toluene was added dropwise at −2°C to 0°C. The reaction mixture was allowed to warm to ambient temperature and after 22 hours, was diluted with water and extracted with ethyl acetate (thrice). The combined organic extracts were washed with water, brine, dried and concentrated *in vacuo*. This product was passed neat under nitrogen at about 40 drops per minute through a pyrolysis column packed with glass helicides heated at 500°C. The pyrolysate was collected in heptane cooled by a dry ice bath. The heptane was removed *in vacuo* and the residue was dissolved in diethyl ether, washed twice with 5% sodium hydroxide, brine, dried ($Na_2SO_4$), concentrated *in vacuo,* and distilled at 80°C (0.08 mm Hg; 11 Pa) to give a crude produce, which was chromatographed on silica gel with ethyl acetate-hexane as eluent. The major component was distilled to give ethyl 3-methyl-1-(1-oxoethyl)-3-cyclopentene-1-carboxylate as a colourless liquid, b.p. 85°C (1.3 mm Hg; 173 Pa).

A solution of potassium hydroxide (4.03 g) in water (20 ml) was added in one portion to a solution of 1.00 g of this product in MeOH (5 ml). The reaction mixture was warmed to reflux for 20 minutes, diluted with water and extracted with diethyl ether three times. The combined extracts were washed with brine, dried ($Na_2SO_4$), concentrated *in vacuo* and vacuum distilled.

A solution of this product in 40 ml of tetrahydrofuran was added to 24 ml methyl magnesium bromide in diethyl ether at −50°C to −10°C under nitrogen. The reaction mixture was kept at −20°C, and after 2 hours was diluted with cold saturated ammonium chloride. The solution was extracted with diethyl ether three times, and the combined extracts were washed with brine, dried ($MgSO_4$) and concentrated *in vacuo*. The residue was separated by flash silica gel chromatography using about 2.4 L of a 40/2/8 hexane/tetrahydrofuran/ethyl acetate mixture. A small portion of the fraction containing the desired product was distilled to give 0.26 g of the desired product as a colourless liquid, b.p. 50°C (0.6 mm Hg; 80 Pa).

Example 11

2,2,4-trimethyl-3-cyclohexen-1-ol

A mixture of 352 g of 1-methoxy-4-methyl-1,4-cyclohexadiene (85% purity), 1350 ml of diethyl ether, 28 g of oxalic acid and 900 ml of water was stirred for 21 hours at 25°C. The aqueous layer was separated and extracted twice with diethyl ether. The combined ether solutions were washed with sodium bicarbonate, dried, concentrated and Claisen-distilled to give 250 g of 4-methyl-3-cyclohexen-1-one, b.p. 63—65°C (13 mm Hg; 1733 Pa). 37.5 g of this product was mixed with 100 ml of diethyl ether, 89.5 g of methyl iodide and 0.3 g of methyltrioctylammonium chloride. This mixture was stirred at ambient temperature and treated with 30 g of granular sodium hydroxide. After 20 minutes at gentle reflux heat was applied to maintain the reflux for 2 hours longer. The cooled mixture was diluted with diethyl ether and treated with water. The ether was separated and the aqueous layer was extracted with ether. The combined ether extracts were washed, dried, concentrated and Claisen-distilled to give 36.5 g of 2,2,4-trimethyl-3-cyclohenen-1-one, b.p. 60°C (10 mm Hg; 1333 Pa). 27.6 g of this ketone in 250 ml of ethanol was added portionwise 7.6 g of sodium borohydride. A cooling bath was used to hold the temperature at 25—30°C. After 2 hours the mixture was

poured into water and extracted three times with methylene chloride. The combined methylene chloride extracts were washed, dried, and concentrated at 25°C and 0.1 mm Hg (13 Pa) to give 27.7 g of the desired product.

Example 12

1,2,2,4-Tetramethyl-3-cyclohexen-1-ol

To a stirred solution of 80 ml of 2.9 M methyl magnesium chloride (in tetrahydrofuran) mixed with 200 ml of dry tetrahydrofuran was added dropwise at 25—30°C a solution of 27.6 g of 2,2,4-trimethyl-3-cyclohexen-1-one in 30 ml of tetrahydrofuran. After 1 hour longer at 25°C and 1 hour at 45—50°C, the mixture was cooled and treated carefully with 50 ml of saturated ammonium sulphate. The mixture was extracted twice with diethyl ether, and the combined ether extracts were dried, concentrated and distilled through a micro Vigreux column to give 12.5 g (*A*), b.p. 115—125°C (100 mm Hg; 13332 Pa), which was mainly unchanged ketone starting material and 11.1 g (*B*), b.p. 125—115°C (100—50 mm Hg; 13332—6666 Pa), which was the desired product of 81% purity. The final cut of 2.0 g (*C*), b.p. 115—120°C (50—20 mm Hg; 6666—2666 Pa) was the desired product of 87% purity.

Examples 13 and 14

These examples described the preparation of ketones useful as precursors for alcohols of the general formula II.

Example 13

1,4-Dimethyl-7-oxabicyclo[2,2,1]heptan-2-one

A 25.6 g portion of nitroethylene was dissolved in diethyl ether and dried ($Na_2SO_4$), and after filtering and rinsing with ether, the filtrate was made up to 250 ml of solution. This solution was added to a stirred, cooled solution of 42.2 g of 2,5-dimethylfuran in 150 ml of diethyl ether in the presence of 0.4 g of hydroquinone. The reaction mixture was refrigerated for several days, stirred at room temperature for 24 hours, treated with charcoal, filtered and stripped to yield 53.3 g of 1,4-dimethyl-5-nitro-7-oxabicyclo[2,2,1]hept-2-ene as an amber oil. This product was placed in a ½ liter Parr bomb with 50 ml of ethanol and 10% palladium on powdered charcoal catalyst. The bomb was charged with hydrogen gas under pressure. After the desired quantity of hydrogen gas had been absorbed, the reaction mixture was removed from the bomb, filtered to remove the catalyst, and stripped. The residue taken up in diethyl ether, washed with water, dried ($MgSO_4$), filtered and stripped to yield 4.8 g of 1,4-dimethyl-2-nitro-7-oxabicyclo[2,2,1]heptane as a brownish oil.

To 900 ml of 0.1 N potassium hydroxide was added with stirring 5.0 g of this product. To this mixture was added 125 ml of 2 M $MgSO_4$ solution and sufficient water to bring the total volume to 2.5 liters, followed dropwise at 0—5°C by a solution of 4.5 g of potassium permanganate in 125 ml of water. After stirring for 1 hour, the resulting mixture was filtered. The filtrate was decolourized with sodium bisulphite and extracted with methylene chloride. The extract was dried ($MgSO_4$), filtered and stripped to give 3.3 g of a pale yellow oil. This procedure was repeated to yield 3.2 g of a pale yellow oil. These products were combined and distilled to yield 5.8 g of the desired product as a colourless oil.

Example 14

1,4,5,6-Tetramethyl-7-oxabicyclo[2,2,1]hept-5-en-2-one

27.8 g of tetramethylfuran and 24.9 g of alpha-acetoxy-acrylonitrile were allowed to stand for one week. A solid product weighing 39.9 g was obtained by decantation, and added to a stirred solution of 20.4 g of sodium hydroxide in 400 ml of ethanol. After 24 hours, the mixture was poured into 1 liter of water and extracted with five 200 ml portions of methylene chloride. The combined methylene chloride extracts were washed, dried, concentrated, and Claisen distilled to give 23.7 g of the desired product, b.p. 63°C (2 mm Hg; 266 Pa).

Example 15 to 25

These Examples describe the preparation of alcohols of the general formula II.

Example 15

(±)-2-*exo*-Hydroxy-1-methyl-4-isopropyl-7-oxabicyclo[2,2,1]heptane

To a solution of 22.3 g of 85% *m*-chloroperbenzoic acid in 150 ml of methylene chloride was added over 40 minutes a solution of 15.4 g of (±)-terpinen-4-ol in 30 ml methylene chloride at a temperature of about 0°C. The reaction mixture was stirred for 20 hours at room temperature, then cooled to 5°C. A solid was filtered and rinsed with cold methylene chloride. The combined filtrates were washed successively with one-eighth saturated potassium carbonate, saturated sodium sulphite, and then water, dried and Claisen distilled to yield 8.9 g of product, b.p. 109—113°C at 8 mm Hg (1067 Pa). Recrystallization of the solidified distillate from pentane gave 5.5 g of the desired product, m.p. 42—58°C.

Example 16

(±)-2-*exo*-Hydroxy-1,4-diethyl-7-oxabicyclo[2,2,1]heptane

To a stirred solution of 15.4 g of 1,4-diethyl-3-cyclohexen-1-ol and 0.4 g of vanadium(IV) bis(2,4-pentanedioate) oxide in 125 ml of methylene chloride was added dropwise at gentle reflux 11.0 g of 90% tert-butyl hydroperoxide. After an additional 2 hours reflux, the mixture was cooled slightly, treated with

7.2 ml of glyme containing 0.58 g of p-toluenesulphonic acid, and refluxed for 2 hours longer. The cooled mixture was stirred for half an hour with 1.0 g of anhydrous sodium acetate and filtered. The filtrate was concentrated and Claisen-distilled to give 14.4 g of product, b.p. 65—78°C (1 mm Hg; 133 Pa).

Example 17

(±)-2-*exo*-Hydroxy-1-methyl-4-(1-methyl-1-(phenylsulphonyl)-ethyl)-7-oxabicyclo[2,2,1]heptane

To a stirred solution of 13.1 g of (±)-4-methyl-1-(1-methyl-1-(phenylthio)ethyl)-3-cyclohexen-1-ol and 0.27 g of vanadium(IV) bis(2,4-pentanedioate) oxide in 130 ml of methylene chloride was added dropwise at reflux 20.0 g of 90% tert-butyl hydroperoxide in 10 minutes. The mixture was refluxed for one hour longer, and after cooling, washed, dried and vacuum-concentrated at 50—55°C. To the resulting residue of about 18 g., 5 ml of glyme containing 0.4 g of *p*-toluenesulphonic acid was added. The mixture was stirred overnight at 5—25°C. 100 ml of chloroform was added and the ether and pentane were removed by vacuum-concentration. The residual chloroform solution was washed with potassium carbonate, dried and concentrated to a residue of 14.9 g. This residue was purified by dry column chromatography using a 30:220:500 mixture of tetrahydrofuran: ethyl acetate:hexane as eluent. This column was divided into 12 equal parts; fraction 10 gave 5 g of product. Recrystallization of fraction 10 from diethyl ether gave 3.0 g of the desired product, m.p. 108—110°C.

Example 18

1,4,5,6-Tetramethyl-7-oxabicyclo[2,2,1]hept-5-ene-2,3-*cis*-diol (*exo*)

A mixture of 4.8 g of vinylene carbonate and 7.0 g of tetramethylfuran were allowed to warm overnight on the steam bath. The resulting dark solution was distilled at 45°C and 0.1 mm to give 9.1 g of a semi-solid mixture of isomers. The mixture was stirred 5 hours with 100 ml of diethyl ether and filtered to give 1.9 g of 1,4,5,6-tetramethyl-7-oxabicyclo[2,2,1]hept-5-en-2,3-cis-*exo*-diol cyclic carbonate, m.p. 164—165°C.

To a stirred solution of 0.6 g of this product in 15 ml of ethanol was added a solution of 0.6 g of sodium hydroxide in 5 ml of water. After 18 hours at 25°C, the mixture was filtered to remove 0.3 g of sodium carbonate and the filtrate was treated with solid carbon dioxide and vacuum-concentrated at 60°C. The residue was boiled with ethyl acetate, and filtered hot, and the filtrate was concentrated to a residue of 0.4 g of the desired *exo* product, m.p. 142—145°C.

Example 19

1,4,5,6-Tetramethyl-7-oxabicyclo[2,2,1]heptane-2,3-*cis*-*exo*-diol

A 6.7 g portion of the unsaturated-*cis*-*exo*-diol cyclic carbonate of Example 18 above was shaken with hydrogen under pressure using 75 ml of absolute ethanol as solvent and 2.0 g of 5% palladium on carbon as catalyst. Hydrogenation was complete in half an hour Vacuum concentration gave a mixture of solid product and catalyst. This was treated with hot acetone and filtered to remove the catalyst. Chilling of the filtrate gave 4.1 g of cyclic carbonate, m.p. 162—166°C.

A solution of 2.5 g of this product in 50 ml of ethanol was treated with a solution of 2.0 g of sodium hydroxide in 15 ml of water to give 2.1 g of the desired product, m.p. 179—183°C without recrystallization.

Example 20

(±)-2-*endo*-Hydroxy-1,4-dimethyl-7-oxabicyclo[2,2,1]heptane

To a solution of 2.3 g of 1,4-dimethyl-7-oxabicyclo[2,2,1]-heptan-2-one in 20 ml of dry tetrahydrofuran was added dropwise 16 ml of boron hydride in tetrahydrofuran under nitrogen atmosphere and at 0—5°C. The resulting mixture was stirred at 0°C for 10 minutes, quenched in water, extracted with methylene chloride, dried ($MgSO_4$), and stripped to give 2.0 g of pale yellow oil. The reaction was repeated to yield 1.0 g of pale yellow oil. The combined products were distilled at 102—105°C (4 mm Hg; 533 Pa) to give 1.6 g of the desired product as a colourless oil.

Example 21

1-Methyl-3,3-diethyl-2-oxabicyclo[2,2,2]octan-6-*exo*-ol

To a stirred solution of 102 g of alpha,alpha-diethyl-4-methyl-3-cyclohexen-1-methanol in 750 ml methylene chloride was added 125 g of 85% *m*-chloroperbenzoic acid portionwise over 1½ hours at 20—23°C. The reaction mixture was stirred for an additional 1½ hours at 20°C, and washed successively with 40 ml aqueous potassium carbonate solution, 30 ml of 2N sodium hydroxide solution, and saturated sodium chloride solution. The organic phase was dried ($MgSO_4$) and stripped to yield 111 g of crude product.

Example 22

1-Methyl-3,3-tetramethylene-2-oxabicyclo[2,2,2]octan-6-*exo*-ol

To 32 g of 1-(4-methyl-3-cyclohexen-1-yl)cyclopentan-1-ol in 40 ml of methylene chloride was added portionwise 39 g of 85% m-chloroperbenzoic acid at 20—24°C. The reaction mixture was then stirred at room temperature overnight. After addition of 1 g of p-toluenesulphonic acid, the reaction mixture was refluxed for 2 hours, cooled to room temperature, washed successively with 200 ml of aqueous potassium carbonate, 200 ml of 2N sodium hydroxide solution, and saturated sodium chloride solution dried with $MgSO_4$, and stripped to yield 31 g of product as an oil.

Example 23

1,3,3-Trimethyl-7-oxabicyclo[2,2,1]heptan-2-*exo*-ol

A solution of 27.7 g of the alcohol of Example 11 above in 250 ml of methylene chloride was treated with 1.0 g of vanadium(IV) bis(2,4-pentanedioate) oxide and 22.0 g of 90% tert-butylhydroperoxide. After stirring overnight at 25°C, the mixture was washed with 1 N sodium hydroxide, dried and Claisen-distilled to give 23.7 g of cis-3,4-epoxy-2,2,4-trimethylcyclohexanol, b.p. 58—61°C (1 mm Hg; 133 Pa).

To a stirred solution of 20.0 g of this epoxy-alcohol in 200 ml of methylene chloride held at 25°C was added dropwise over 20 min a solution of 0.4 g of *p*-toluenesulphonic acid in 5 ml of glyme. After 1 hour longer, the solution was washed with dilute potassium carbonate, dried, concentrated, and Claisen-distilled to give 11.1 g, b.p. 55—77°C (1.0—0.1 mm Hg; 133—13 Pa). This was redistilled to give 2.8 g of the desired product, b.p. 77—80°C (3.0 mm Hg; 400 Pa).

Example 24

1,5-Dimethyl-8-oxabicyclo[3,2,1]octan-6-*exo*-ol

A mixture of zinc/silver couple (2.23 g) and 2,5-dimethylfuran (25 ml) in dry tetrahydrofuran (35 ml) was cooled at −10°C. A solution of α,α,α',α'-tetrabromoacetone in dry tetrahydrofuran (15 ml) was added dropwise over a period of 3 hours with stirring, and the resulting mixture was allowed to warm to room temperature with stirring for an additional 16.5 hours. The insoluble materials were removed by filtration and the filtrate was concentrated. The resulting tarry, brown residue was eluted through a Florisil (Trade Mark) column with diethyl ether to give an orange solid. Further purification by flash-column chromatography produced 2,4-dibromo-1,5-dimethyl-8-oxabicyclo[3,2,1]oct-6-ene-3-one (3.58 g) as a colourless solid, m.p. 115—20°C.

To a stirred slurry of this product (18.0 g) in a saturated methanolic solution of ammonium chloride (514 ml) was added portionwise zinc/copper couple (51 g) over 30 minutes at 23°C. The suspension was stirred for an additional 2 hours and filtered. The filtrate was diluted with a saturated $Na_2H_2$-ethylenediaminetetraacetic acid solution (400 ml). Extraction with methylene chloride (5 × 100 ml) and drying ($MgSO_4$) produced, upon evaporative removal of solvent, the desired ketone (9.0 g). Distillation afforded 1,5-dimethyl-8-oxabicyclo[3,2,1]oct-6-en-3-one (7.3 g) as colourless crystals, m.p. 62—63°C.

To a stirred suspension of lithium aluminium hydride (4.6 g of a 57% oil suspension) in dry diethyl ether (300 ml) was added a solution of this product (10.0 g) in diethyl ether (50 ml) over 0.5 hour at 20°C. The reaction mixture was held for 2 hours at 20°C, cooled to 0°C, and washed successively with water (2.5 ml), 15% aqueous sodium hydroxide (2.5 ml) and water (7.5 ml), and filtered. The filter cake was washed with diethyl ether, and the combined ether filtrates were dried ($MgSO_4$), filtered and concentrated to give 13.0 g of oil. The oil was eluted through a column of Florisil (Trade Mark) first with hexane and then diethyl ether. The ether fractions were combined and concentrated to give 1,5-dimethyl-8-oxabicyclo[3,2,1]oct-6-en-3-ol (10.0 g) as a 66:34 mixture of epimers.

para-Toluenesulphonyl chloride (15.5 g) was added portionwise over 20 minutes to an ice-cold stirred solution of this product (10.0 g) in dry pyridine (75 ml). The reaction mixture was stirred at 0°C for 0.5 hour, allowed to stand in a refrigerator (0°C) for 72 hours, and poured into a mixture of 80 ml concentrated hydrochloric acid and 100 g of ice. After stirring for 10 minutes, the precipitated solid was filtered. The collected solid dissolved methylene chloride was dried ($MgSO_4$), filtered and concentrated to give 17.6 g (88%) of the p-toluenesulphonate ester, m.p. 75—77°C, as a 70:30 mixture of epimers.

A stirred, ice-cold solution of this ester (6.16 g) in dry tetrahydrofuran (80 ml) was treated with a 1 M solution of Super Hydride in tetrahydrofuran (80 ml) dropwise over 30 minutes. The mixture was allowed to warm slowly to room temperature and then heated at reflux overnight. The reaction mixture was cooled to 0°C and treated over 5 minutes with ethyl iodide (4.8 ml). After warming to 23°C and holding for 1 hour at 23°C, the mixture was recooled to 0°C. To the resulting mixture a 1 M solution of borane in tetrahydrofuran (40 ml) was added over a 20 minute period. The mixture was then allowed to warm to 23°C and held for an additional 3.5 hours. The mixture was cooled to 0°C and water (24 ml) was slowly added, followed by dropwise addition of a 3 N aqueous sodium hydroxide solution (32 ml), and then a 30% hydrogen peroxide solution (32 ml). After warming to 23°C, the mixture was maintained at this temperature overnight and subsequently warmed to 40°C for 1 hour to complete the oxidation step. Aqueous workup was followed by flash column chromatography ($SiO_2$) to give the desired product (1.68 g) as a water-white oil.

Example 25

*Endo* and *Exo* 1-methyl-3,3-tetramethylene-2-oxabicyclo-[2,2,2]-octan-6-ol

This Example illustrates a method for the interconversion of isomers of compounds of the general formula II.

22.1 g of oxalyl chloride was stirred in 375 ml of methylene chloride, and 29.7 g of dimethyl sulphoxide in 75 ml of methylene chloride was added over five minutes while maintaining the temperature at −60°C. The mixture was stirred for 10 minutes and 31 g of the product of Example 21 above in 160 ml of methylene chloride was added over five minutes while maintaining the temperature at −60°C. The reaction mixture was stirred for 15 minutes and 80 g of triethylamine was added over ten minutes at −60°C. The reaction mixture was allowed to warm to room temperature, and then 475 ml of water was added. The resulting methylene chloride phase was separated, and washed successively with dilute hydrochloric acid, saturated

sodium bicarbonate solution, and saturated sodium chloride solution. The organic phase was dried with $MgSO_4$, and stripped to yield 31 g of an amber oil. Distillation of the oil gave 12.3 g of 1-methyl-3,3-tetramethylene-2-oxabicyclo[2,2,2]octan-6-one, b.p. 93—98°C (1 mm Hg; 133 Pa).

This product was dissolved in 50 ml dimethylformamide and 50 ml tert-butyl alcohol, and 2.4 g of sodium borohydride was added portionwise over 10 minutes while maintaining the temperature at 20°C with water cooling.

The reaction mixture was stirred at room temperature for 5 hours, stored overnight under refrigeration, then quenched in 90 ml of water, stripped of solvents and extracted with methylene chloride. The organic phase was washed with saturated sodium chloride solution, dried with $MgSO_4$, and stripped to yield 12 g of oil. Distillation of the oil gave 10.4 g of product, b.p. 93—8°C (0.4 mm Hg; 53 Pa) as a mixture of *exo* and *endo* isomers.

Examples 26 to 32

These examples describe the preparation of compounds of the general formula I.

Example 26

(±)-2-*exo*-Benzyloxy-1-methyl-4-isopropyl-7-oxabicyclo[2,2,1]heptane

To a solution of 1.7 g of (±)-2-*exo*-hydroxy-1-methyl-4-isopropyl-7-oxabicyclo[2,2,1]heptane in 15 ml of dimethylformamide was added at room temperature 0.5 g of 50% sodium hydride. The resulting mixture was stirred overnight at room temperature, heated for one-half hour at 50°C, cooled to room temperature, and after 1.5 g of benzyl chloride was added in one portion, stirred at room temperature for three hours, heated to 50°C for one hour, cooled, poured into 50 ml of water, and extracted with one 50 ml and two 25 ml portions of methylene chloride. The combined methylene chloride extracts were washed with 100 ml of water, dried and evaporated to give an orange oil. Claisen distillation yielded 1.5 g of the desired product, b.p. 103°C at 0.08 mm Hg (11 Pa).

Example 27

(±)-2-*exo*-Benzyloxy-1,4-diethyl-7-oxabicyclo[2,2,1]heptane

A stirred mixture of 3.8 g of crude (±)-2-*exo*-hydroxy-1,4-diethyl-7-oxabicyclo[2,2,1]heptane, 50 ml. of N,N-dimethylacetamide and 1.0 g of sodium hydride (washed with n-hexane) was warmed slowly to 80°C to complete hydrogen evolution. The cooled mixture was treated with 3.0 g of benzyl chloride, again warmed to 80°C, and after ½ hour, poured into ice water, and extracted twice with methylene chloride. The combined organic extracts were washed, dried, concentrated and Claisen-distilled to give 2.6 g of the desired product, b.p. 105—110° (0.1 mm Hg; 13 Pa).

Example 28

*endo*-1,3,3-Trimethyl-6-(2-fluorobenzyloxy)-2-oxabicyclo[2,2,1]heptane

A solution of 0.50 g of *endo*-1,3,3-trimethyl-6-hydroxy-2-oxabicyclo[2,2,1]heptane in 5 ml of dimethylformamide was added to a 60% oil dispersion of 0.17 g of sodium hydride in 2 ml of dimethylformamide at 10°C under nitrogen. After 45 minutes, 0.73 g of 2-fluorobenzyl bromide in 2 ml of dimethylformamide was added, and after 13.5 hours, a trace of potassium iodide. After 15 hours more, the reaction mixture was diluted with brine, dried ($MgSO_4$) and concentrated *in vacuo*. The residue was purified by silica gel flash chromatography using 1 l of 90/4/6 hexane/tetrahydrofuran/ethyl acetate as eluent and vacuum distilled to give 0.41 g of the desired product as a colourless liquid, b.p. 85°C at 0.05 mm (7 Pa).

Example 29

(±)-2-*exo*-(2-Methylbenzyloxy)-1-methyl-4-isopropyl-7-oxabicyclo[2,2,1]heptane

A 12 l flask was charged under nitrogen with 264 g of 50% sodium hydride followed by 3 l of dry dimethylformamide. The resulting mixture was heated to 60°C and a solution of 850 g of (±)-2-*exo*-hydroxy-1-methyl-4-isopropyl-7-oxabicyclo[2,2,1]heptane in 1.5 of dimethylformamide was added over 3 hours while maintaining the reaction mixture at 60—70°C. Then the reaction mixture was cooled to 20°C and 730.6 g of 2-methylbenzyl chloride was added over 1½ hours while cooling the reaction mixture to 20—25°C. The reaction mixture was stirred at room temperature for 16 hours. The resulting mixture was poured into 20 l of water, acidified with concentrated hydrochloric acid and extracted three times with 3.5 l of hexane. The combined extracts were washed with 3 l of water, dried ($MgSO_4$), filtered and evaporated to dryness to yield 1280 g of the desired product.

Example 30

1-Methyl-3,3-diethyl-6-*endo*-benzyloxy-2-oxabicyclo[2,2,2]octane

A solution of 3.5 g of crude 1-methyl-3,3-diethyl-2-oxabicyclo-[2,2,2]octan-6-*endo*-ol in 25 ml of tetrahydrofuran was added dropwise to a slurry of 1.2 g of 47% sodium hydride at 20—40°C, resulting in slow evolution of gas. The reaction mixture was heated at reflux for 2 hours, then cooled to room temperature, and 0.7 g of tetrabutylammonium iodide was added followed by 3.9 g of benzyl bromide in 30 ml of tetrahydrofuran. The resulting mixture was stirred for 3 days at room temperature, quenched in

water, and extracted with diethyl ether. The ether phase was washed with water and saturated sodium chloride solution, dried with $MgSO_4$, and stripped to give 5.5 g of oil. After distilling most of the low boiling materials, the remaining product was chromatographed on a silica gel column and eluted with methylene chloride-pentane (40—60%) to yield three fractions: 0.3 g 1,2-diphenylethene, 1.1 g, primarily the desired *endo*-isomer-rich product, and 1.2 g, primarily the *exo* isomer of the desired product. Rechromatography of the second fraction yielded 0.7 g of pure *endo* form of the desired product.

Example 31

2-*exo*-benzyloxy-4-(benzyloxycarbonylmethyl)-1-methyl-7-oxabicyclo[2,2,1]heptane.

To a stirred mixture of 26.0 g of zinc dust, 2.0 g of iodine and 40 ml of benzene was added rapidly at 65—75°C a solution of 22.0 g of 4-methyl-3-cyclohexen-1-one and 62.6 g of ethyl bromoacetate in 400 ml of benzene. After 5 hours at reflux the mixture was cooled to 10°C and treated dropwise with 300 ml of 10% acetic acid. After 15 minutes, the layers were separated and the aqueous layer was extracted twice with 150 ml of benzene. The combined organic layers were washed successively with water, sodium bicarbonate solution and water. After drying and concentration, Claisen distillation gave 33.0 g of 1-(ethoxycarbonylmethyl)-4-methyl-3-cyclohexen-1-ol, b.p. 82—84°C (0.5 mm Hg; 67 Pa).

To a stirred, refluxing solution of 46.2 g of this alcohol and 1.2 g of vanadium (IV) bis(2,4-pentanedioate) oxide in 400 ml of methylene chloride was added dropwise 25.3 g of 90% tert-butyl hydroperoxide. After 2 hours longer at reflux, the solution was cooled, dried over magnesium sulphate, and filtered. The stirred filtrate was treated with 12 ml of glyme containing 1.0 g of p-toluenesulphonic acid. After 18 hours at 25°C, the mixture was washed with dilute carbonate and dried. Vacuum-concentration at 50°C gave 52.3 g of dark amber oil. GLC analysis indicated the presence of 56% of desired product. Chromatographic purification using ethyl acetate as eluent gave 16.8 g of 4-(ethoxycarbonylmethyl)-2-*exo*-hydroxy-1-methyl-7-oxabicyclo[2,2,1]heptane as an oil.

To a stirred solution of 4.3 g of this oil in 15 ml of ethanol was added a solution of 0.9 g of sodium hydroxide in 3 ml of water. After 24 hours at 25°C, the mixture was vacuum-concentrated at 30°C. The residue was dissolved in 30 ml of N,N-dimethylacetamide and treated with 1.0 g of 50% sodium hydride. After 6 hours at 25°C, 5.5 g of benzyl chloride was added, and stirring was continued for 4 days. The mixture was poured into water, extracted 3 times with methylene chloride, and the combined methylene chloride extracts were washed, dried and concentrated to a residue of 8.1 g. This oil was purified by column chromatography to give 2.6 g of desired product as an oil.

Example 32

1,3,3-Trimethyl-6-*endo*-7-*syn*-bis-benzyloxy-2-oxabicyclo[2,2,2]octane

To a stirred solution of 2.5 g of 1,3,3-trimethyl-2-oxabicyclo[2,2,2]octane-6-*endo*-7-*syn*-diol (Tetrahedron, *27,* 753 (1971)) in 50 ml of N,N-dimethylacetamide was added 0.7 g of 50% sodium hydride. After one hour at 25—85°C, the mixture was cooled and treated with 1.7 g of benzyl chloride. After 30 minutes at 50—60°C, the mixture was cooled and poured into water. Extraction with methylene chloride, followed by Claisen distillation, gave 1.2 g of 1,3,3-trimethyl-7-*endo*-(benzyloxy)-2-oxabicyclo[2,2,2]octan-6-*syn*-ol, b.p. 135—150°C (0.2 mm Hg; 27 Pa). The residue from the distillation was recrystallized from hexane to give 0.6 g of the desired product, m.p. 99—101°C.

Examples 33 to 99

By methods analogous to those described in Examples 26 to 32, further compounds of the general formula I were prepared. Details are given in Table I, in which the compounds are described with reference to formula I(a):

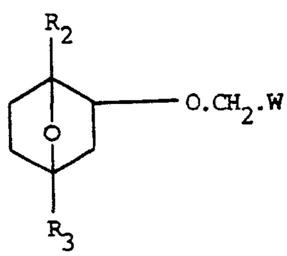

I (a)

TABLE I

| Example No. | $R_2$ | In the general formula (Ia) $R_3$ | W | Rotation and Configuration | Boiling Point °C (mm Hg; Pa) |
|---|---|---|---|---|---|
| 33 | $CH_3$ | i-$C_3H_7$ | phenyl | (−) *exo* | 100—110 (0.5; 67) |
| 34 | $CH_3$ | i-$C_3H_7$ | 2-methyl-phenyl | (±) *exo* | 110—113 (0.1; 13) |
| 35 | $CH_3$ | i-$C_3H_7$ | 2-methyl-phenyl | (−) *exo* | 114—116 (0.1; 13) |
| 36 | $CH_3$ | i-$C_3H_7$ | 2-pyridyl | (±) *exo* | 108—112 (0.05; 7) |
| 37 | $CH_3$ | i-$C_3H_7$ | 2-pyridyl | (−) *exo* | 114 (0.1; 13) |
| 38 | $CH_3$ | i-$C_3H_7$ | 2-chloro-phenyl | (±) *exo* | 114—115 (0.05; 7) |
| 39 | $CH_3$ | i-$C_3H_7$ | 2-fluorophenyl | (±) *exo* | 105—112 (0.05; 7) |
| 40 | $CH_3$ | i-$C_3H_7$ | 2-fluorophenyl | (−) *exo* | 103—105 (0.1; 13) |
| 41 | $CH_3$ | i-$C_3H_7$ | 2,6-dichloro-phenyl | (±) *exo* | 125—127 (0.05; 7) |
| 42 | $C_2H_5$ | $CH_3$ | phenyl | (±) *exo* | 95—98 (0.05; 7) |
| 43 | $C_2H_5$ | $C_2H_5$ | 2-methyl-phenyl | (±) *exo* | 110—112 (0.1; 13) |
| 44 | $C_2H_5$ | $C_2H_5$ | 2-fluorophenyl | (±) *exo* | 101—103 (0.1; 13) |
| 45 | $C_2H_5$ | $C_2H_5$ | 2-chlorophenyl | (±) *exo* | 118—120 (0.1; 13) |
| 46 | $C_2H_5$ | $C_2H_5$ | 2-pyridyl | (±) *exo* | 110—111 (0.1; 13) |
| 47 | $CH_3$ | $C_2H_5$ | phenyl | (±) *exo* | 98—99 (0.5; 67) |
| 48 | $CH_3$ | $C_2H_5$ | 2-methyl-phenyl | (±) *exo* | 115 (0.3; 40) |
| 49 | $C_2H_5$ | $CH_3$ | 2-methylphenyl | (±) *exo* | 110—112 (0.15; 20) |
| 50 | $CH_3$ | $C_2H_5$ | 2-chlorophenyl | (±) *exo* | 113—114 (0.1; 13) |
| 51 | $C_2H_5$ | $CH_3$ | 2-chlorophenyl | (±) *exo* | 112—115 (0.05; 7) |
| 52 | $CH_3$ | $C_2H_5$ | 2,6-dichloro-phenyl | (±) *exo* | 128—130 (0.1; 13) |

TABLE I (continued)

| Example No. | $R_2$ | In the general formula (Ia) $R_3$ | W | Rotation and Configuration | Boiling Point °C (mm Hg; Pa) |
|---|---|---|---|---|---|
| 53 | $C_2H_5$ | $CH_3$ | 2,6-dichloro-phenyl | (±) *exo* | 130—132 (0.05; 7) |
| 54 | $CH_3$ | i-$C_3H_7$ | 2-chlorophenyl | (−) *exo* | 112 (0.05; 7) |
| 55 | $CH_3$ | $CH_3$ | phenyl | (±) *exo* | 93 (0.05; 7) |
| 56 | $CH_3$ | $CH_3$ | 2-methyl-phenyl | (±) *exo* | 102—104 (09.05; 7) |
| 57 | $C_2H_5$ | $C_2H_5$ | 2,6-dichloro phenyl | (±) *exo* | 134—138 (0.2; 27) |
| 58 | $CH_3$ | i-$C_3H_7$ | 2,6-dichloro-phenyl | (±) *exo* | 145—147 (<1; <133) |
| 59 | $CH_3$ | $CH_3$ | 2,6-dichloro phenyl | (±) *exo* | 119—120 (0.05; 7) |
| 60 | $CH_3$ | i-$C_3H_7$ | phenyl | (+) *exo* | 107 (0.2; 27) |
| 61 | $CH_3$ | i-$C_3H_7$ | 2-fluorophenyl | (±) *endo* | 105—108 (0.2; 27) |
| 62 | $CH_3$ | i-$C_3H_7$ | 2-methyl-phenyl | (+) *exo* | 109—112 (0.1; 13) |
| 63 | $CH_3$ | i-$C_3H_7$ | 2-pyridyl | (+) *exo* | 118—120 (0.1; 13) |
| 64 | $CH_3$ | i-$C_3H_7$ | 2-fluorophenyl | (+) *exo* | 118 (<1; <133) |
| 65 | $CH_3$ | i-$C_3H_7$ | 2,5-dimethyl-phenyl | (+) *exo* | 120—125 (0.1; 13) |
| 66 | $CH_3$ | n-$C_4H_9$ | phenyl | (±) *exo* | 115—116 (0.1; 13) |
| 67 | n-$C_3H_7$ | $CH_3$ | phenyl | (±) *exo* | 107—110 (0.2; 27) |
| 68 | $CH_3$ | phenyl | phenyl | (±) *exo* | 155 (0.15; 20) |
| 69 | $CH_3$ | i-$C_3H_7$ | 2-methyl-phenyl | (±) *endo* | 115—118 (0.2; 27) |
| 70 | $CH_3$ | 1-methyl-1-phenyl-sulphonyl-ethyl | phenyl | (±) exo | solid: m. pt. 94—96°C |

TABLE I (continued)

| Example No. | $R_2$ | In the general formula (Ia) $R_3$ | W | Rotation and Configuration | Boiling Point °C (mm Hg; Pa) |
|---|---|---|---|---|---|
| 71 | $CH_3$ | 1-methyl-1-methyl-sulphonyl-ethyl | phenyl | (±) exo | amber oil |
| 72 | $CH_3$ | 1-chloro-1 methylethyl | phenyl | (±) exo | 120—122 (0.15; 20) |
| 73 | $CH_3$ | 1-cyano-1-methylethyl | phenyl | (±) exo | 139—140 (0.1; 13) |
| 74 | $CH_3$ | 1-cyano-1-methylethyl | 2,6-dichlorophenyl | (±) exo | 162—165 (0.15; 20) |
| 75 | $CH_3$ | 1-hydroxy-1-methyl-ethyl | phenyl | (±) exo | 114—115 (0.1; 13) |
| 76 | $CH_3$ | 1-methoxy-1-methylethyl | phenyl | (±) exo | 110—115 (0.1; 13) |
| 77 | $CH_3$ | 1-ethoxy-1-methylethyl | phenyl | (±) exo | 120—125 (0.2; 27) |
| 78 | $CH_3$ | 1-isopropo-xy-1-methyl-ethyl | phenyl | (±) exo | 120—130 (0.2; 27) |
| 79 | $CH_3$ | i-$C_3H_7$ | ethynyl | (±) exo | 100—105 (4; 533) |
| 80 | $CH_3$ | i-$C_3H_7$ | 4-fluoro-phenyl | (±) exo | 102—105 (0.1; 13) |
| 81 | $CH_3$ | 1-chloro-1-methylethyl | 2-chlorophenyl | (±) exo | not measured |
| 82 | $CH_3$ | benzyl | phenyl | (±) exo | 140—150 (0.1; 13) |
| 83 | $CH_3$ | $CH_3$ | phenyl | (±) endo | not measured |
| 84 | $CH_3$ | i-$C_3H_7$ | 3-fluoro-phenyl | (±) exo | 103—104 (0.1; 13) |
| 85 | $CH_3$ | i-$C_3H_7$ | 2-furyl | (±) exo | 94—95 (0.2; 27) |
| 86 | $CH_3$ | 1-chloro-1-methyl-ethyl | 2-fluoro-phenyl | (±) exo | 133—134 (0.2; 27) |
| 87 | $CH_3$ | 1-chloro-1-methyl-ethyl | 2-methyl-phenyl | (±) exo | 128—130 (0.1; 13) |

TABLE I (continued)

| Example No. | In the general formula (Ia) $R_2$ | $R_3$ | W | Rotation and Configuration | Boiling Point °C (mm Hg; Pa) |
|---|---|---|---|---|---|
| 88 | $CH_3$ | $C_2H_5$ | 2-fluoro-phenyl | (±) exo | 95—97 (0.1; 13) |
| 89 | $CH_3$ | i-$C_3H_7$ | phenyl | (±) endo | 103 (0.15; 20) |
| 90 | $CH_3$ | H | phenyl | (±) exo | 100—101 (0.2; 27) |
| 91 | $CH_3$ | $CH_2{=}CH{-}CH_2$ | phenyl | (±) exo | 109—115 (0.1; 13) |
| 92 | $CH_3$ | phenyl | 2-fluoro-phenyl | (±) exo | not measured |
| 93 | $CH_3$ | phenyl | 2-methyl-phenyl | (±) exo | not measured |
| 94 | n-$C_6H_{13}$ | $CH_3$ | phenyl | (±) exo | 125—128 (0.1; 13) |
| 95 | $CH_3$ | i-$C_3H_7$ | α-naphthyl | (±) exo | 150—164 (0.15; 20) |
| 96 | $CH_3$ | i-$C_3H_7$ | pyrazinyl | (±) exo | 115—125 (0.1; 13) |
| 97 | $CH_3$ | i-$C_3H_7$ | CN | (±) exo | 103—105 (1.0; 133) |
| 98 | $CH_3$ | i-$C_3H_7$ | 2-pyrida-zinyl | (±) exo | solid: m.pt 105—108 |
| 99 | (deleted) | | | | |

Examples 100 to 139

By methods analogous to those described in Examples 26 to 32, further compounds of the general formula I were prepared. All the compounds had the general formula Ia given above, in which $R^2$ is a methyl group; $R^3$ is an isopropyl group; and W is a substituted phenyl group. Table II gives the details, describing the position and nature of the substituent on the phenyl group W. All compounds were (±) exo isomers.

TABLE II

| Example No. | Substituent on Phenyl Group W in Formula Ia | Boiling Point (°C (mm Hg; Pa) |
|---|---|---|
| 100 | 3-Cl | 128—135 (0.15; 20) |
| 101 | 4-Cl | 128—134 (0.15; 20) |
| 102 | 3,4-$Cl_2$ | 140—149 (0.15; 20) |
| 103 | 2,4-$Cl_2$ | 138—150 (0.15; 20) |
| 104 | 3-$CF_3$ | 112—117 (0.15; 20) |
| 105 | 3-$CH_3$ | 115—122 (0.15; 20) |
| 106 | 4-$CH_3$ | 117—124 (0.15; 20) |
| 107 | 2-$OCH_3$ | 130—137 (0.15; 20) |
| 108 | 4-$OCH_3$ | 126—134 (0.15; 20) |
| 109 | 2,5-$Cl_2$ | 129—137 (0.1; 13) |
| 110 | 2-F, 6-Cl | 116—124 (0.1; 13) |
| 111 | 3-$OCH_3$ | 126—131 (0.1; 13) |
| 112 | 3,5-$Cl_2$ | 132—137 (0.1; 13) |
| 113 | 2,6-$(CH_3)_2$ | 111—115 (0.1; 13) |
| 114 | 2-Br | 128—135 (0.1; 13) |
| 115 | 2-$CF_3$ | 105—110 (0.1; 13) |
| 116 | 4-$CF_3$ | 107—113 (0.1; 13) |
| 117 | 3-$NO_2$ | 150—153 (0.1; 13) |
| 118 | 2,6-$F_2$ | 115—120 (0.2; 27) |
| 119 | 2-CN | 140—148 (0.15; 20) |
| 120 | 2-$CONH_2$ | 180—184 (0.2; 27) |
| 121 | 2,6-$(OCH)_3$ | 147—150 (0.25; 33) |
| 122 | 2-COOH | m.p. 96—108 |
| 123 | 2-$OC_2H_5$ | 132—137 (0.15; 20) |
| 124 | (deleted) | |
| 125 | 2-$CH_2NH_2$ | 138—140 (0.1; 13) |
| 126 | 2-$NHCH_3$ | 130 (0.1; 13) |
| 127 | 2-$N(CH_3)C_2H_5$ | 130—140 (0.15; 20) |
| 128 | (deleted) | |
| 129 | 2-$SCH_3$ | 143—146 (0.15; 20) |

TABLE II (continued)

| Example No. | Substituent on Phenyl Group W in Formula Ia | Boiling Point (°C (mm Hg; Pa) |
|---|---|---|
| 130 | 2-S(O)$CH_3$ | m.p. 108—110 |
| | 2 Isomers | |
| 131 | 2-S(O)$CH_3$ | |
| 132 | 2-$OCH_2$Ph | 173—175 (0.1; 13) |
| 133 | 2-OH | 137—138 (0.1; 13) |
| 134 | (deleted) | |
| 135 | 4-Br | 140—143 (0.1; 13) |
| 136 | 4-CN | 142—145 (0.1; 13) |
| 137 | (deleted) | |
| 138 | (deleted) | |
| 139 | 2-I | 141—144 (0.2; 27Pa) |

Examples 140 to 166

By method analogous to those described in Examples 26 to 32, further compounds of the general formula I were prepared. Details are given in Table III, in which the compounds are described with reference to formula I(b):

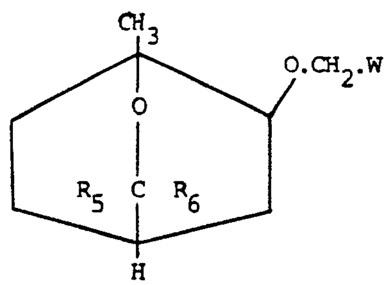

I (b)

TABLE III

In the General Formula I(b)

| Example No. | $R_5$ | $R_6$ | W | b.p., °C (mm Hg; Pa) |
|---|---|---|---|---|
| 140 | $CH_3$ | $CH_3$ | 2-fluorophenyl | 112—118 (0.1; 13) |
| 141 | $CH_3$ | $CH_3$ | 2-chlorophenyl | 129—134 (0.2; 27) |
| 142 | $CH_3$ | $CH_3$ | 2-methylphenyl | 108—112 (0.15; 20) |
| 143 | $CH_3$ | $CH_3$ | 2,6-dichlorophenyl | m.p. 100—102 |
| 144 | $CH_3$ | $CH_3$ | 2-pyridyl | 112—113 (0.15; 20) |
| 145 | $C_2H_5$ | $C_2H_5$ | 2,6-dichlorophenyl | m.p. 118—119 |
| 146 | $C_2H_5$ | $C_2H_5$ | 2-chlorophenyl | LIQUID* |
| 147 | $C_2H_5$ | $C_2H_5$ | 2-fluorophenyl | LIQUID* |
| 148 | $C_2H_5$ | $C_2H_5$ | 2-methylphenyl | LIQUID* |

TABLE III (continued)

In the General Formula I(b)

| Example No. | $R_5$ | $R_6$ | W | b.p., °C (mm Hg; Pa) |
|---|---|---|---|---|
| 149 | $C_2H_5$ | $C_2H_5$ | 2-pyridyl | LIQUID* |
| 150 | $—(CH_2)_4—$ | | 2-fluorophenyl | LIQUID* |
| 151 | $CH_3$ | $CH_3$ | 2,6-difluorophenyl | 106—110 (0.15; 20) |
| 152 | $—(CH_2)_5—$ | | phenyl | LIQUID* |
| 153 | $—(CH_2)_5—$ | | 2-fluorophenyl | LIQUID* |
| 154 | $—(CH_2)_4—$ | | 2-methylphenyl | LIQUID* |
| 155 | $—(CH_2)_5—$ | | 2-methylphenyl | LIQUID* |
| 156 | $C_2H_5$ | $C_2H_5$ | 2,5-dimethylphenyl | LIQUID* |
| 157 | $CH_3$ | $CH_3$ | 2-methoxyphenyl | 128—130 (0.15; 20) |
| 158 | $C_2H_5$ | $C_2H_5$ | 2,4-dimethylphenyl | LIQUID* |
| 159 | $C_2H_5$ | $C_2H_5$ | 2,6-difluorophenyl | LIQUID* |
| 160 | $C_2H_5$ | $C_2H_5$ | 2,6-dimethylphenyl | m.p. 68—70 |
| 161 | $C_2H_5$ | $C_2H_5$ | 2-ethylphenyl | LIQUID* |
| 162 | $CH_3$ | $CH_3$ | phenyl | 95 (0.1; 13) |
| 163 | $CH_3$ | $CH_3$ | ethynyl | 92—97 (2.5; 333) |
| 164 | $CH_3$ | $CH_3$ | 2-ethylphenyl | 113—115 (0.05; 7) |
| 165 | $CH_3$ | $CH_3$ | 2-methylphenyl | 108—112 (0.15; 20) |
| 166 | $—(CH_2)_4—$ | | phenyl | LIQUID* |

* Isolated by column chromatography; boiling point not determined.

Examples 167 to 181

By methods analogous to those described in Examples 26 to 32, the following compounds of the general formula I were prepared.

Example 167

6-*exo*-(2-methylbenzyloxy)-1,5-dimethyl-8-oxobicyclo[3,2,1]octane, a water-white oil.

Example 168

6-*exo*-(2-fluorobenzoyloxy)-1,5-dimethyl-8-oxabicyclo[3,2,1]octane, a water white oil.

Example 169

*endo*-1,3,3-trimethyl-6-(2-methylbenzyloxy)-2-oxabicyclo[2,2,1]heptane, boiling point 100°C (0.06 mm; 8 Pa).

Example 170

*endo*-1,3,3-trimethyl-6-benzyloxy-2-oxabicyclo[2,2,1]heptane, boiling point 70°C (0.05 mm Hg; 7 Pa).

Example 171

(−)-2-*exo*-(2-methylbenzoyloxy)-1-methyl-4-isopropyl-7-oxabicyclo[2,2,1]heptane.

Example 172 (c.f. Example 181)

2-Benzyloxy-1,4,5,6-tetramethyl-7-oxabicyclo[2,2,1]hept-5-ene, *endo*:*exo* ratio 76:24.

Example 173

2-*exo*-Benzyloxy-1,3,3-trimethyl-7-oxabicyclo[2,2,1]heptane, boiling point 97—99°C (0.1 mmHg; 13 Pa).

Example 174

2-*exo*-Benzyloxy-1,3,3,4-tetramethyl-7-oxabicyclo[2,2,1]heptane, boiling point 95—100°C (0.2 mm Hg; 27 Pa).

Example 175

2-*exo*-Benzyloxy-1,3,3,4,5,5-hexamethyl-7-oxabicyclo[2,2,1]heptane, boiling point 110—115°C (0.1 mmHg; 13 Pa).

Example 176

2-*endo*-Benzyloxy-1,2-dimethyl-4-isopropyl-7-oxabicyclo[2,2,1]heptane, boiling point 106—110°C (0.15 mm Hg; 20 Pa).

Example 177

(−)-1,3,3-trimetyl-6-*endo*-benzyloxy-2-oxabicyclo[2,2,2]octane, melting point 48—50°C, $[alpha]_D$ −75.4° ($CHCl_3$).

Example 178

2-*exo*-benzyloxy-1,4,5,6-tetramethyl-7-oxabicyclo[2,2,1]hept-5-en-3-*exo*-ol, boiling point 130—135°C (0.1 mm Hg; 13 Pa).

Example 179

2-*exo*-benzyloxy-3-*exo*-methoxy-1,4,5,6-tetramethyl-7-oxabicyclo[2,2,1]heptane, boiling point 123—133°C (0.2 mm Hg; 27 Pa).

Example 180

1,3,3-trimethyl-6-*syn*-methoxy-7-*endo*-benzyloxy-2-oxabicyclo[2,2,2]octane, boiling point 130—150°C (0.1 mm Hg; 13 Pa).

Example 181

2-benzyloxy-1,4,5,6-tetramethyl-7-oxabicyclo[2,2,1]hept-5-ene, endo:exo ratio 76:24. Distillation was used to produce product mixtures with various *endo*:*exo* ratios e.g. compound 181(a), *endo*:*exo* 91:7, compound 181(b), *endo*:*exo* 15:85.

Example 182 to 193

These examples illustrate methods for the conversion of one compound of the general formula I into a different compound of the general formula I.

Examples 182 to 184

The 4-(1-chloro-1-methylethyl) compounds of Examples 72, 86 and 87 were each treated with 1.1 molar equivalents of sodium hydride in N,N-dimethylacetamide at 80°C for 2 hours to give the corresponding dehydrochlorinated products.

Example 182

(±)-2-*exo*-benzyloxy-4-(1-methylethenyl)-1-methyl-7-oxabiccylo[2,2,1]heptane, boiling point 110—114°C (0.1 mm Hg; 13 pa).

Example 183

(±)-2-*exo*-(2-fluorobenzyloxy)-4-(1-methylethenyl)-1-methyl-7-oxabicyclo[2,2,1]heptane, boiling point 105°C (0.1 mm Hg; 13 Pa).

Example 184

(±)-2-*exo*-(2-methylbenzyloxy)-4-(1-methylethenyl)-1-methyl-7-oxabicyclo[2,2,1]heptane, boiling point 110—114°C (0.1 mm Hg; 13 Pa).

Example 185

2-Benzyloxy-1,4,5,6-tetramethyl-7-oxabicyclo[2,2,1]heptane

a) To a 500 ml Parr hydrogenation vessel were charged 0.9 g of 2-benzyloxy-1,4,5,6-tetramethyl-7-oxabicyclo[2,2,1]hept-5-ene, *endo*:*exo* ratio 15:85, 50 ml of ethanol, 0.3 g of 5% palladium on carbon and 2 ml of triethylamine. After shaking at 25°C for 4 hours, hydrogen absorption ceased. The catalyst was removed by filtration, and the filtrate was vacuum-concentrated to give 0.8 g of 2-*exo*-benzyloxy-1,4,5,6-tetramethyl-7-oxabicyclo[2,2,1] heptane as a colourless oil, b.p. 135 (0.1 mm Hg; 13 Pa).

b) In a similar manner, the *endo* ether was prepared from a starting material having and endo:exo ratio 97:7, and had a b.p. of 97—99°C (0.15 mm Hg; 20 Pa).

Example 186

2-*exo*-Benzyloxy-3-*exo*-methoxy-1,4,5,6-tetramethyl-7-oxabicyclo[2,2,1]hept-5-ene

To a stirred mixture of 3.6 g of the ether of Example 178 above, and 50 ml of dimethylformamide was added 0.6 g of 60% sodium hydride. Heat was applied to raise the temperature to 80°C over a 1 hour period. After cooling to 20°C, 3 ml of methyl iodide was added. This caused a temperature rise to 35°C. Stirring was continued at ambient temperature for half an hour and the solution was worked up to give 3.0 g of the desired product, b.p. 120°C (0.1 mm Hg; 13 pa).

Example 187

2-*exo*-Benzyloxy-5,6-epoxy-3-*exo*-methoxy-1,4,5,6-tetramethyl-7-oxabicyclo[2,2,1]heptane

To a cold, stirred solution of 1.3 g of the ether of Example 186 above in 25 ml of methylene chloride was added 1.0 g of 85% *m*-chloroperbenzoic acid. After 2 hours at 5—25°C, the mixture was washed with dilute potassium carbonate containing dilute sodium sulphite, dried and concentrated under vacuum at 75°C to a constant weight of 1.3 g of the desired product.

Example 188

2-*exo*-Benzyloxy-4-carboxymethyl-1-methyl-7-oxobicyclo[2,2,1]heptane

To a stirred solution of 3.1 g of ether of Example 31 above in 15 ml of ethanol was added a solution of 0.6 g of sodium hydroxide in 2.5 ml of water. After 29 hours at 25°C, the mixture was acidified using 6N hydrochloric acid. After dilution with 150 ml portions of methylene chloride, the organic layer was washed with water. The dried methylene chloride solution was concentrated to a residue of 2.8 g. This was purified by plate chromatography to give 1.7 g of the desired product as an oil.

Example 189

(±)-2-*exo*-Benzyloxy-1-methyl-4-acetyl-7-oxabicyclo[2,2,1]heptane

To a stirred mixture of 15.1 g of the ether of Example 182 above, 150 ml of diethyl ether and 150 ml of water were added 30 ml of 2% osmium tetroxide in t-butanol. After 15 mintues, 27.2 g of sodium metaperiodate was added and the reaction was continued at reflux for 18 hours. The ether layer was separated and the aqueous layer was extracted with ether. The combined ether extracts were washed, dried, concentrated, and Claisen-distilled to give 11.1 g of the desired product, b.p. 120°C (0.1 mmHg; 13 Pa).

Example 190

(±)-2-*exo*-Benzyloxy-1-methyl-4-carboxy-7-oxabicyclo[2,2,1]heptane

To a stirred solution of 8.4 g of the ether of Example 189 above in 240 ml of dioxane held at 10—15°C was added a solution of 6.5 ml of bromine in 160 ml of water containing 42 g of potassium hydroxide. The mixture was stirred for 18 hours at 10—25°C and then poured into 300 ml of one-fourth saturated sodium bisulphite. This mixture was first extracted with 300 ml of diethyl ether and then acidified with 110 ml of 6 N hydrochloric acid. The acidic product was extracted into three 250 ml portions of diethyl ether. The combined other extracts were washed, dried and concentrated at 40°C and $<1$ mm Hg ($<133$ Pa) to give 8.8 g of crude product. Recrystallization from ether-pentane gave 6.5 g of the desired product, m.p. 93—95°C.

Example 191

(±)-2-*exo*-Benzyloxy-1-methyl-4-carbamoyl-7-oxabicyclo[2,2,1]heptane

The product of Example 190 above was concentrated to the acid chloride and the latter was treated with ammonia to give the desired product, m.p. 122—123°C.

Example 192

(±)-*exo*-Benzyloxy-1-methyl-4-cyano-7-oxabicyclo[2,2,1]heptane

The ether of Example 191 above was treated with acetic anhydride and pyridine to give the desired product, recovered as an oil.

Example 193

(±)-2-*exo*-(Cyclohexylmethoxy)-1-methyl-4-isopropyl-7-oxabicyclo[2,2,1]heptane

A 7.8 g sample of the product of Example 26 above was dissolved in 40 ml of methanol containing 0.5 ml of acetic acid and treated with 1.5 g of 5% rhodium on alumina. This was shaken at 25°C with hydrogen at an initial pressure of 50 psig ($3.4 \times 10^5$ Pa). After 4 hours, the catalyst was removed by filtration and the filtrate was distilled to give 7.4 g of the desired product, b.p. 97—101°C (0.1 mm Hg; 13 pa).

Example 194

Herbicidal Activity

In this example, the species of plants that were tested were;

Barnyardgrass (watergrass) — *Enchinochloa crus-galli*

Large crabgrass — *Digitaria sanguinalis*

Downy brome — *Bromus tectorum*
Yellow foxtail — *Setaria lutescens*
Redroot pigweed — *Amaranthus retroflexus*
Sicklepod — *Cassia obtusifolia*
Velvetleaf — *Abutilon theophrasti*
Garden cress — *Lepidium sativum*
Johnsongrass — *Sorghum halepense*

The preemergence (soil) herbicidal activity of compounds of the invention was evaluated by planting seeds of barnyardgrass, garden cress, downy brome, velvetleaf, yellow foxtail, and sicklepod in test tubes, nominally measuring 25 × 200 millimeters, filled about three-quarters full of untreated soil, in each case covered on top with about 2.5 cubic centimeters of soil treated with a certain amount of the test compound. The treated soil applied to the tubes containing the barnyard grass and cress seeds contained one milligram of the test compound per tube, and contained 0.1 milligram of the test compound per tube containing the seeds of the other plants. These dosages were approximately twenty and two pounds of test compound per acre, respectively. The seeds were planted on top of the treated soil and covered with about 1.5 cubic centrimetres of untreated soil.

The planted soil was held under controlled conditions of temperature, moisture, and light for 9 to 10 days. The amounts of germination and growth in each tube were evaluated on a 0 to 9 scale, the numeric ratings having the following meanings:

| Rating | Meaning |
|---|---|
| 9 | No living tissue |
| 8 | Plant severely damaged and expected to die |
| 7 | Plant badly damaged, but expected to live |
| 6 | Moderate plant damage, but complete recovery expected |
| 5 | Intermediate damage (probably unacceptable damage for crop plants) |
| 3—4 | Observable damage |
| 1—2 | Plant slightly affected, possibly by the chemical, possibly due to biological variability |
| 0 | No visible effect |

The postemergence (foliar) herbicidal activity of compounds of the invention was evaluated by spraying 10 day old large crabgrass plants, 13 day old redroot pigweed plants, 6 day old Johnsongrass plants, 9 day old velvetleaf plants, 9 day old yellow foxtail plants and 9 day old sicklepod plants to runoff with a liquid formulation of the test compound. The crabgrass and pigweed plants were sprayed with 2.4 milliliters of a 0.25% solution (about ten pounds of the test compound per acre), and other plants were sprayed with 2.4 milliliters of a 0.025% solution (about one pound of the test compound per acre). The sprayed plants were held under controlled conditions of temperature, moisture and light for 7 to 8 days and the effect of the test compound was then evaluated visually, the results being rated on the 0 to 9 scale described above.

Results of the preemergence and postemergence herbicidal activity tests conducted on the compounds of the invention are given in Table IV below.

TABLE IV HERBICIDAL ACTIVITY

| Compound of Example No. | Preemergence | | | | | | Postemergence | | | | | |
|---|---|---|---|---|---|---|---|---|---|---|---|---|
| | Barn-yard Grass | Garden Cress | Downy Brome | Velvet Leaf | Yellow Foxtail | Sickle-pod | Crab Grass | Pig Weed | Johnson-grass | Velvet Leaf | Yellow Foxtail | Sickle-pod |
| 26 | 9 | 7 | 9 | 7 | 9 | 7 | 8 | 6 | 8 | 5 | 9 | 4 |
| 60 | 9 | 7 | 9 | 6 | 8 | 5 | 7 | 7 | 4 | 3 | 4 | 2 |
| 89 | 9 | 7 | 9 | 7 | 8 | 3 | 8 | 5 | 4 | 3 | 7 | 3 |
| 34 | 9 | 6 | 9 | 6 | 9 | 6 | 8 | 4 | 4 | 6 | 8 | 4 |
| 35 | 9 | 7 | 9 | 7 | 8 | 6 | 8 | 6 | 6 | 6 | 7 | 6 |
| 39 | 9 | 8 | 9 | 7 | 9 | 7 | 8 | 4 | 7 | 5 | 8 | 3 |
| 64 | 9 | 7 | 9 | 6 | 8 | 3 | 6 | 5 | 0 | 3 | 2 | 2 |
| 40 | 9 | 8 | 9 | 7 | 8 | 7 | 8 | 7 | 8 | 6 | 8 | 6 |
| 41 | 8 | 7 | 8 | 7 | 9 | 6 | 8 | 5 | 3 | 6 | 8 | 3 |
| 38 | 9 | 7 | 9 | 7 | 9 | 5 | 9 | 5 | 3 | 4 | 8 | 3 |
| 36 | 9 | 7 | 9 | 7 | 8 | 6 | 3 | 2 | 0 | 0 | 0 | 0 |
| 63 | 8 | 7 | 6 | 5 | 0 | 2 | 3 | 2 | 0 | 3 | 2 | 2 |
| 37 | 9 | 7 | 9 | 7 | 8 | 4 | 7 | 5 | 3 | 5 | 3 | 2 |
| 27 | 9 | 7 | 9 | 7 | 8 | 7 | 7 | 4 | 6 | 5 | 8 | 3 |
| 43 | 9 | 7 | 9 | 7 | 8 | 7 | 8 | 3 | 6 | 6 | 9 | 2 |
| 44 | 9 | 7 | 9 | 7 | 8 | 7 | 8 | 6 | 5 | 4 | 9 | 2 |
| 46 | 9 | 7 | 9 | 7 | 9 | 7 | 8 | 5 | 0 | 4 | 2 | 2 |

TABLE IV (continued)

| Compound of Example No. | Preemergence | | | | | | Postemergence | | | | | |
|---|---|---|---|---|---|---|---|---|---|---|---|---|
| | Barn-yard Grass | Garden Cress | Downy Brome | Velvet Leaf | Yellow Foxtail | Sickle-pod | Crab Grass | Pig Weed | Johnson-grass | Velvet Leaf | Yellow Foxtail | Sickle-pod |
| 45 | 9 | 7 | 9 | 7 | 8 | 7 | 8 | 6 | 5 | 6 | 8 | 6 |
| 42 | 9 | 8 | 9 | 7 | 9 | 7 | 4 | 4 | 0 | 3 | 2 | 2 |
| 47 | 9 | 8 | 9 | 7 | 8 | 7 | 6 | 5 | 3 | 2 | 6 | 2 |
| 48 | 9 | 7 | 9 | 7 | 8 | 6 | 7 | 6 | 2 | 3 | 5 | 3 |
| 49 | 9 | 7 | 9 | 7 | 8 | 8 | 7 | 5 | 2 | 4 | 7 | 2 |
| 50 | 9 | 7 | 9 | 7 | 8 | 7 | 8 | 5 | 4 | 4 | 7 | 3 |
| 51 | 9 | 7 | 9 | 7 | 9 | 7 | 7 | 6 | 3 | 6 | 5 | 3 |
| 53 | 9 | 7 | 9 | 7 | 8 | 7 | 7 | 6 | 4 | 7 | 7 | 4 |
| 52 | 9 | 7 | 9 | 7 | 9 | 6 | 8 | 7 | 5 | 7 | 7 | 3 |
| 76 | 9 | 7 | 9 | 5 | 8 | 5 | 2 | 2 | 1 | 2 | 1 | 1 |
| 55 | 9 | 7 | 9 | 7 | 8 | 6 | 3 | 2 | 0 | 2 | 0 | 2 |
| 56 | 9 | 7 | 9 | 7 | 8 | 7 | 6 | 3 | 0 | 4 | 2 | 2 |
| 57 | 9 | 6 | 8 | 5 | 8 | 4 | 8 | 5 | 6 | 6 | 8 | 3 |
| 58 | 9 | 7 | 9 | 7 | 8 | 6 | 8 | 5 | 3 | 7 | 7 | 2 |
| 59 | 9 | 7 | 9 | 7 | 8 | 7 | 7 | 4 | 3 | 6 | 4 | 5 |
| 77 | 9 | 7 | 9 | 7 | 8 | 5 | 7 | 3 | 0 | 2 | 0 | 0 |
| 78 | 9 | 7 | 9 | 6 | 8 | 3 | 8 | 6 | 2 | 3 | 2 | 3 |

TABLE IV (continued)

| Compound of Example No. | Preemergence | | | | | | Postemergence | | | | | |
|---|---|---|---|---|---|---|---|---|---|---|---|---|
| | Barn-yard Grass | Garden Cress | Downy Brome | Velvet Leaf | Yellow Foxtail | Sickle-pod | Crab Grass | Pig Weed | Johnson-grass | Velvet Leaf | Yellow Foxtail | Sickle-pod |
| 75 | 9 | 7 | 9 | 7 | 9 | 5 | 4 | 3 | 4 | 3 | 2 | 2 |
| 65 | 9 | 7 | 9 | 5 | 8 | 3 | 7 | 6 | 6 | 4 | 7 | 4 |
| 54 | 9 | 7 | 9 | 7 | 8 | 4 | 8 | 6 | 8 | 6 | 7 | 3 |
| 74 | 8 | 7 | 9 | 7 | 7 | 3 | 6 | 6 | 3 | 6 | 2 | 0 |
| 66 | 9 | 7 | 9 | 7 | 8 | 6 | 9 | 7 | 8 | 5 | 7 | 3 |
| 68 | 9 | 7 | 8 | 7 | 8 | 5 | 2 | 2 | 2 | 7 | 7 | 3 |
| 72 | 9 | 7 | 9 | 6 | 8 | 5 | 9 | 3 | 8 | 6 | 8 | 2 |
| 70 | 7 | 3 | 5 | 2 | 5 | 2 | 5 | 5 | 2 | 5 | 0 | 2 |
| 67 | 9 | 7 | 9 | 6 | 9 | 6 | 8 | 6 | 4 | 6 | 7 | 3 |
| 71 | 8 | 3 | 4 | 3 | 7 | 3 | 2 | 2 | 0 | 2 | 0 | 2 |
| 79 | 9 | 7 | 9 | 5 | 5 | 2 | 2 | 0 | 0 | 2 | 0 | 2 |
| 80 | 9 | 7 | 9 | 7 | 8 | 2 | 8 | 6 | 8 | 6 | 7 | 3 |
| 90 | 9 | 7 | 9 | 7 | 8 | 7 | 8 | 5 | 0 | 3 | 7 | 3 |
| 82 | 8 | 7 | 3 | 3 | 0 | 0 | 8 | 7 | 0 | 2 | — | 0 |
| 84 | 9 | 7 | 9 | 6 | 8 | 3 | 8 | 4 | 5 | 4 | 7 | 4 |
| 85 | 9 | 6 | 9 | 6 | 8 | 6 | 7 | 2 | 5 | 4 | 6 | 2 |
| 86 | 9 | 6 | 9 | 6 | 8 | 6 | 8 | 3 | 7 | 6 | 7 | 2 |

TABLE IV (continued)

| Compound of Example No. | Preemergence | | | | | | Postemergence | | | | | |
|---|---|---|---|---|---|---|---|---|---|---|---|---|
| | Barn-yard Grass | Garden Cress | Downy Brome | Velvet Leaf | Yellow Foxtail | Sickle-pod | Crab Grass | Pig Weed | Johnson-grass | Velvet Leaf | Yellow Foxtail | Sickle-pod |
| 87 | 9 | 6 | 9 | 6 | 8 | 6 | 8 | 6 | 5 | 6 | 7 | 3 |
| 88 | 9 | 7 | 9 | 6 | 8 | 6 | 8 | 7 | 4 | 3 | 8 | 4 |
| 33 | 9 | 8 | 9 | 7 | 9 | 7 | 8 | 7 | 5 | 4 | 7 | 2 |
| 91 | 9 | 7 | 9 | 7 | 8 | 6 | 7 | 6 | 0 | 4 | 6 | 2 |
| 61 | 8 | 7 | 9 | 7 | 8 | 6 | 3 | 2 | 0 | 2 | 0 | 2 |
| 69 | 8 | 7 | 8 | 6 | 8 | 5 | 6 | 2 | 2 | 3 | 2 | 2 |
| 81 | 9 | 7 | 7 | 7 | 8 | 6 | 8 | 6 | 7 | 6 | 8 | 6 |
| 92 | 9 | 6 | 9 | 5 | 7 | 5 | 8 | 7 | 7 | 6 | — | 6 |
| 93 | 7 | 6 | 8 | 4 | 7 | 4 | 8 | 7 | 7 | 6 | — | 5 |
| 140 | 9 | 7 | 9 | 6 | 8 | 6 | 8 | 3 | 3 | 3 | 7 | 5 |
| 141 | 9 | 6 | 9 | 6 | 8 | 5 | 8 | 4 | 3 | 5 | 5 | 5 |
| 142 | 9 | 7 | 9 | 6 | 8 | 6 | 8 | 6 | 3 | 6 | 7 | 4 |
| 143 | 8 | 6 | 9 | 6 | 7 | 3 | 8 | 7 | 5 | 7 | 7 | 7 |
| 144 | 9 | 7 | 9 | 5 | 8 | 6 | 6 | 4 | 0 | 5 | 3 | 3 |
| 151 | 9 | 7 | 9 | 7 | 8 | 7 | 8 | 5 | 6 | 4 | 7 | 7 |
| 157 | 9 | 7 | 6 | 6 | 8 | 5 | 7 | 3 | 0 | 3 | 0 | 0 |
| 30 | 9 | 5 | 9 | 3 | 8 | 0 | 9 | 4 | 5 | 6 | 8 | 7 |

TABLE IV (continued)

| Compound of Example No. | Preemergence | | | | | | Postemergence | | | | | |
|---|---|---|---|---|---|---|---|---|---|---|---|---|
| | Barn-yard Grass | Garden Cress | Downy Brome | Velvet Leaf | Yellow Foxtail | Sickle-pod | Crab Grass | Pig Weed | Johnson-grass | Velvet Leaf | Yellow Foxtail | Sickle-pod |
| 177 | 9 | 7 | 9 | 5 | 8 | 6 | 6 | 5 | 0 | 2 | 5 | 3 |
| 146 | 8 | 0 | 9 | 0 | 6 | 0 | 8 | 6 | 6 | 7 | 8 | 3 |
| 147 | 9 | 7 | 9 | 2 | 8 | 3 | 8 | 7 | 6 | 6 | 7 | 6 |
| 148 | 9 | 7 | 9 | 0 | 8 | 3 | 8 | 7 | 3 | 6 | 7 | 3 |
| 149 | 9 | 7 | 9 | 4 | 8 | 4 | 8 | 7 | 0 | 4 | 3 | 6 |
| 166 | 9 | 6 | 9 | 5 | 8 | 3 | 9 | 7 | 7 | 6 | 7 | 3 |
| 150 | 9 | 6 | 9 | 5 | 8 | 3 | 8 | 6 | 5 | 4 | 7 | 2 |
| 154 | 8 | 6 | 6 | 4 | 8 | 2 | 8 | 7 | 7 | 6 | 7 | 5 |
| 152 | 9 | 7 | 8 | 5 | 8 | 5 | 9 | 7 | 7 | 6 | 8 | 7 |
| 153 | 9 | 7 | 9 | 2 | 8 | 3 | 8 | 7 | 7 | 6 | 8 | 7 |
| 155 | 8 | 6 | 5 | 2 | 4 | 2 | 9 | 7 | 8 | 6 | 8 | 7 |
| 158 | 7 | 2 | 3 | 0 | 4 | 0 | 7 | 7 | 4 | 4 | 8 | 5 |
| 159 | 9 | 7 | 9 | 5 | 8 | 5 | 8 | 6 | 6 | 3 | 8 | 6 |
| 145 | 5 | 0 | 4 | 0 | 3 | 0 | 8 | 4 | 2 | 6 | 8 | 7 |
| 162 | 9 | 6 | 9 | 5 | 9 | 6 | 5 | 5 | 3 | 0 | 2 | 2 |
| 161 | 8 | 3 | 7 | 2 | 8 | 3 | 8 | 5 | 4 | 3 | 5 | 7 |
| 163 | 9 | 7 | 9 | 3 | 8 | 4 | 0 | 0 | 0 | 0 | 0 | 0 |

TABLE IV (continued)

| Compound of Example No. | Preemergence: Barn-yard Grass | Garden Cress | Downy Brome | Velvet Leaf | Yellow Foxtail | Sickle-pod | Postemergence: Crab Grass | Pig Weed | Johnson-grass | Velvet Leaf | Yellow Foxtail | Sickle-pod |
|---|---|---|---|---|---|---|---|---|---|---|---|---|
| 165 | 9 | 7 | 8 | 7 | 8 | 4 | 7 | 4 | 0 | 3 | 3 | 2 |
| 164 | 9 | 7 | 9 | 6 | 8 | 4 | 8 | 5 | 5 | 6 | 8 | 4 |
| 182 | 9 | 7 | 9 | 6 | 8 | 6 | 9 | 4 | 5 | 4 | 7 | 5 |
| 93 | 9 | 6 | 7 | 5 | 7 | 0 | 7 | 3 | 3 | 5 | 7 | 4 |
| 101 | 8 | 6 | 8 | 5 | 5 | 2 | 8 | 4 | 6 | 6 | 7 | 3 |
| 102 | 0 | 0 | 0 | 0 | 0 | 0 | 7 | 3 | 3 | 4 | 7 | 0 |
| 103 | 6 | 0 | 3 | 0 | 0 | 0 | 7 | 3 | 5 | 5 | 7 | 2 |
| 95 | 6 | 5 | 4 | 3 | 4 | 2 | 7 | 4 | 3 | 6 | 7 | 3 |
| 104 | 7 | 2 | 0 | 0 | 0 | 0 | 3 | 0 | 2 | 2 | 3 | 0 |
| 105 | 9 | 6 | 8 | 6 | 8 | 5 | 7 | 4 | 6 | 5 | 7 | 4 |
| 106 | 9 | 6 | 7 | 5 | 6 | 5 | 8 | 3 | 7 | 5 | 8 | 3 |
| 107 | 9 | 6 | 9 | 6 | 8 | 3 | 9 | 6 | 7 | 6 | 7 | 4 |
| 108 | 8 | 5 | 5 | 5 | 5 | 2 | 7 | 4 | 2 | 3 | 2 | 3 |
| 109 | 6 | 8 | 5 | 0 | 5 | 0 | 8 | 5 | 6 | 4 | 7 | 2 |
| 110 | 9 | 7 | 9 | 6 | 8 | 6 | 8 | 7 | 6 | 6 | 8 | 6 |
| 111 | 8 | 7 | 7 | 6 | 6 | 4 | 8 | 6 | 3 | 3 | 5 | 5 |
| 112 | 0 | 0 | 0 | 0 | 0 | 0 | 7 | 3 | 3 | 2 | 3 | 2 |

TABLE IV (continued)

| Compound of Example No. | Preemergence Barn-yard Grass | Garden Cress | Downy Brome | Velvet Leaf | Yellow Foxtail | Sickle-pod | Postemergence Crab Grass | Pig Weed | Johnson-grass | Velvet Leaf | Yellow Foxtail | Sickle-pod |
|---|---|---|---|---|---|---|---|---|---|---|---|---|
| 114 | 9 | 7 | 8 | 6 | 8 | 2 | 9 | 7 | 6 | 6 | 7 | 3 |
| 115 | 9 | 9 | 8 | 5 | 7 | 2 | 8 | 3 | 3 | 3 | 7 | 4 |
| 116 | 3 | 2 | 0 | 0 | 0 | 2 | 3 | 3 | 2 | 2 | 0 | 2 |
| 113 | 8 | 7 | 9 | 6 | 8 | 6 | 7 | 4 | 3 | 6 | 7 | 2 |
| 118 | 9 | 7 | 9 | 6 | 8 | 6 | 8 | 3 | 6 | 5 | 7 | 2 |
| 96 | 9 | 7 | 9 | 6 | 8 | 4 | 2 | 3 | 1 | 2 | 0 | 2 |
| 184 | 9 | 7 | 9 | 6 | 8 | 5 | 8 | 3 | 7 | 6 | 7 | 2 |
| 183 | 9 | 7 | 9 | 6 | 8 | 5 | 9 | 3 | 7 | 6 | 7 | 2 |
| 119 | 9 | 7 | 9 | 6 | 8 | 0 | 7 | 3 | 2 | 5 | 7 | 2 |
| 97 | 2 | 7 | 0 | 0 | 0 | 0 | 0 | 0 | 0 | 0 | 0 | 0 |
| 94 | 5 | 0 | 0 | 0 | 0 | 0 | 4 | 3 | 2 | 3 | 0 | 2 |
| 120 | 9 | 7 | 4 | 6 | 6 | 3 | 1 | 3 | 0 | 2 | 1 | 2 |
| 121 | 9 | 7 | 6 | 4 | 6 | 3 | 3 | 4 | 0 | 3 | 0 | 3 |
| 122 | 9 | 7 | 0 | 0 | 0 | 0 | 0 | 2 | 0 | 0 | 0 | 0 |
| 98 | 3 | 3 | 0 | 0 | 0 | 0 | 1 | 2 | 1 | 0 | 0 | 0 |
| 186 | 9 | 3 | 7 | 0 | 6 | 0 | 3 | 4 | 0 | 2 | 0 | 0 |
| 178 | 5 | 3 | 0 | 2 | 0 | 0 | 0 | 3 | 0 | 0 | 0 | 0 |

TABLE IV (continued)

| Compound of Example No. | Preemergence | | | | | | Postemergence | | | | | |
|---|---|---|---|---|---|---|---|---|---|---|---|---|
| | Barn-yard Grass | Garden Cress | Downy Brome | Velvet Leaf | Yellow Foxtail | Sickle-pod | Crab Grass | Pig Weed | Johnson-grass | Velvet Leaf | Yellow Foxtail | Sickle-pod |
| 187 | 9 | 7 | 8 | 4 | 8 | 4 | 3 | 0 | 0 | 0 | 0 | 0 |
| 179 | 9 | 7 | 6 | 2 | 6 | 4 | 2 | 2 | 0 | 2 | 0 | 2 |
| 187 | 8 | 7 | 0 | 0 | 0 | 0 | 1 | 2 | 0 | 0 | 0 | 0 |
| 181a | 7 | 3 | 0 | 2 | 0 | 2 | 2 | 6 | 0 | 0 | 0 | 0 |
| 181b | 8 | 3 | 7 | 2 | 4 | 4 | 3 | 5 | 1 | 2 | 2 | 3 |
| 185a | 9 | 7 | 8 | 4 | 7 | 0 | 6 | 4 | 1 | 3 | 1 | 2 |
| 185b | 7 | 4 | 5 | 0 | 0 | 0 | 2 | 3 | 0 | 2 | 0 | 2 |
| 123 | 9 | 8 | 4 | 0 | 6 | 0 | 4 | 0 | 0 | 0 | 0 | 0 |
| 126 | 8 | 6 | 7 | 2 | 3 | 2 | 4 | 3 | 1 | 3 | 1 | 3 |
| 127 | 7 | 2 | 3 | 2 | 3 | 0 | 3 | 3 | 2 | 3 | 1 | 2 |
| 128 | 7 | 5 | 0 | 0 | 0 | 0 | 0 | 3 | 0 | 2 | 0 | 0 |
| 129 | 9 | 6 | 6 | 5 | 7 | 2 | 7 | 4 | 3 | 3 | 6 | 2 |
| 173 | 9 | 7 | 9 | 2 | 7 | 4 | 3 | 5 | 0 | 0 | 0 | 0 |
| 174 | 9 | 3 | 4 | 0 | 3 | 0 | 0 | 0 | 0 | 0 | 0 | 0 |
| 31 | 7 | 3 | 3 | 3 | 0 | 5 | 2 | 5 | 1 | 3 | 0 | 2 |
| 130 | 8 | 5 | 2 | 3 | 1 | 2 | 2 | 3 | 2 | 3 | 1 | 2 |
| 131 | 7 | 6 | 2 | 3 | 2 | 2 | 2 | 8 | 0 | 3 | 0 | 2 |

TABLE IV (continued)

| Compound of Example No. | Preemergence | | | | | | Postemergence | | | | | |
|---|---|---|---|---|---|---|---|---|---|---|---|---|
| | Barn-yard Grass | Garden Cress | Downy Brome | Velvet Leaf | Yellow Foxtail | Sickle-pod | Crab Grass | Pig Weed | Johnson-grass | Velvet Leaf | Yellow Foxtail | Sickle-pod |
| 117 | 7 | 5 | 5 | 3 | 0 | 3 | 3 | 3 | 0 | 3 | 0 | 3 |
| 168 | 9 | 7 | 9 | 3 | 9 | — | 7 | 5 | 2 | 2 | 2 | 2 |
| 167 | 9 | 7 | 8 | 6 | 8 | 4 | 7 | 5 | 2 | 4 | 6 | 4 |
| 28 | 9 | 7 | 9 | 7 | 8 | 7 | 7 | 5 | 3 | 3 | 0 | 0 |
| 169 | 9 | 7 | 9 | 7 | 8 | 7 | 7 | 6 | 0 | 6 | 0 | 2 |
| 170 | 9 | 7 | 9 | 7 | 7 | 7 | 8 | 3 | 4 | 4 | 0 | 0 |

**Claims for the Contracting States: BE CH DE FR GB IT LI LU NL SE**

1. A compound of the general formula I

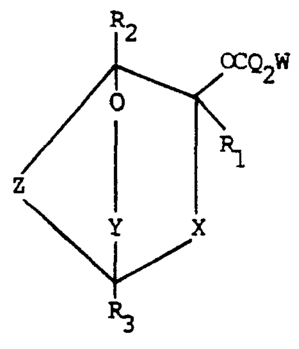

(I)

wherein

X is $-CR_4R_4-$;

Y is $(-CR_5R_6-)_n$ in which n is 0 or 1;

Z is $(-CR_7R_7-)_p$ in which p is 1, 2 or 3;

$R_1$ is a hydrogen atom or $C_{1-6}$ alkyl group optionally substituted by up to 3 fluorine, chlorine and/or bromine atoms;

$R_2$ is a hydrogen atom or a $C_{1-6}$ straight-chain alkyl group;

$R_3$ is a hydrogen atom; a cyano group; a $C_{1-10}$ alkyl group; a $C_{1-6}$ alkyl group which is substituted by one or more halogen atoms or by a hydroxy group, a cyano group, a $C_{1-6}$ alkoxy group, a phenoxy group, a $C_{1-6}$ alkylsulphonyl group, a phenylsulphonyl group, a benzylsulphonyl group, an azido group, a $C_{1-6}$ alkoxycarbonyl group, a benzyloxycarbonyl group, a hydroxycarbonyl group, a phosphoryl group, a phosphoryloxy group, or an amine oxide, carbamoyl or thiocarbamoyl group in which each nitrogen is optionally substituted by 1 or 2 $C_{1-4}$ alkyl groups; a $C_{2-4}$ alkenyl or alkynyl group; an aryl or aralkyl group, each containing from 6 to 11 carbon atoms including 1 to 4 carbon atoms in any alkyl portion, optionally ring substituted by one or more fluorine, chlorine and/or bromine atoms or by a $C_{1-2}$ alkyl or alkoxy group, each optionally substituted by one or more fluorine and/or chlorine atoms; a group $-CSNH_2$; a group $-CO_2R_8$ or $-CON(R_8)_2$ in which $R_8$ is a hydrogen atom or a $C_{1-6}$ alkyl group; or an acetyl group or an oxime or acetal derivative of said group;

each $R_4$ is independently a hydrogen atom; a $C_{1-6}$ alkyl group optionally substituted by up to 3 halogen atoms; a hydroxy group; or a $C_{1-4}$ alkoxy group; or one of $R_4$ and $R_1$ together form a carbon-carbon bond;

$R_5$ and $R_6$ each independently is a hydrogen atom or a $C_{1-2}$ alkyl group; or when located on a carbon atom adjacent to the ring oxygen atom then $R_5$ and $R_6$ may together form an alkylene group containing 4 or 5 carbon atoms;

each $R_7$ independently is a hydrogen atom or a $C_{1-4}$ alkyl group optionally substituted by up to 3 halogen atoms; or when n is 0 then $R_7$ may also be a chlorine or bromine atom, or two of $R_7$ when located on adjacent carbon atoms together form an epoxide ring or a carbon-carbon bond; or when n is 1, then one $R_7$ on the carbon adjacent to the carbon bearing $R_2$ is a hydroxy group, a $C_{7-11}$ aralkoxy group, or a $C_{1-4}$ alkoxy group, and the other $R_7$ is a hydrogen atom;

both of Q are hydrogen atoms or fluorine atoms; and

W represents a cyano group; a $C_{2-4}$ alkenyl or alkynyl group; a pyrimidinyl, pyrazinyl, pyridazinyl, pyridyl, furyl, naphthyl, imidazolyl, triazolyl, thiadiazolyl, 2-quinolinyl, 1-isoquinolinyl, pyrrolyl, N-methylimidazolyl, N-methylpyrazolyl, isoxazolyl, oxazolyl, isothiazolyl, thiazolyl, thienyl or 5-methyl-2-furyl group; or a cyclohexenyl group, or a cycloalkyl group having up to 6 carbon atoms, optionally substituted by a $C_{1-3}$ alkyl group; or a $C_{3-10}$ secondary alkyl group; or a phenyl group which is unsubstituted or substituted by one or more of the following moieties: hydroxy, cyano, nitro, halogen, $C_{1-3}$ alkoxy, haloalkoxy, alkylthio, haloalkylthio, alkylsulphinyl, haloalkylsulphinyl or alkylsulphonyl; alkenyl or alkynyl of up to 4 carbon atoms; phenyl, phenoxy, benzyl or benzyloxy; amino, aminocarbonyl and carboxyl in each of which hydrogen may be placed by $C_{1-4}$ alkyl; and $C_{1-3}$ alkyl which is itself optionally substituted by one or more moieties independently selected from halogen, hydroxy, $C_{1-4}$ alkylthio, $C_{1-4}$ alkoxy, and amino.

2. A compound as claimed in claim 1, in which each $R_4$ independently represents a hydrogen atom or a methyl or ethyl group.

3. A compound as claimed in either claim 1 or claim 2, in which each of $R_5$ and $R_6$ independently represents a hydrogen atom or a methyl or ethyl group.

4. A compound as claimed in any one of claims 1 to 3, in which each $R_7$ independently represents a hydrogen atom or a methyl or ethyl group, or when n is 0, a chlorine or bromine atom, or when n is 1, one $R_7$ on the carbon atom adjacent to the carbon atom bearing $R_2$ is a hydroxy, methoxy, ethoxy or benzyloxy group, the or each other $R_7$ being a hydrogen atom.

5. A compound as claimed in claim 4, in which each $R_7$ represents a hydrogen atom.

6. A compound as claimed in any one of claims 1 to 5, in which n is 0 and p is 2.

7. A compound as claimed in any one of claims 1 to 5, in which n is 1 and p is 2.

8. A compound as claimed in any one of claims 1 to 7, in which W represents a $C_{2-4}$ alkenyl or alkynyl group; a 4-pyrimidinyl, 2-pyrazinyl, 3-pyridazinyl, 2-pyridyl or 2-furyl group; or a phenyl group optionally substituted by one or more of halogen, cyano, amino, $C_{1-3}$ alkoxy or alkylthio each optionally substituted by one or more fluorine and/or chlorine atoms, or $C_{1-2}$ alkyl optionally substituted by one or more fluorine and/or chlorine atoms or by hydroxy, $C_{1-2}$ alkoxy or $C_{1-2}$ alkylthio.

9. A compound as claimed in claim 8, in which W represents an ethynyl group; a 2-pyridyl group; or a phenyl group which is unsubstituted or substituted by one or two substituents selected from methyl groups, fluorine atoms and chlorine atoms.

10. A compound as claimed in any one of claims 1 to 9, in which $R_1$ represents a methyl group or a hydrogen atom.

11. A compound as claimed in any one of claims 1 to 10, in which $R_2$ represents a methyl or ethyl group.

12. A compound as claimed in any one of claims 1 to 11, in which $R_3$ represents a hydrogen atom; a $C_{1-6}$ alkyl group optionally substituted by up to 3 fluorine, chlorine and/or bromine atoms or by a hydroxy, cyano, $C_{1-6}$ alkoxy, $C_{1-6}$ alkylsulphonyl, phenylsulphonyl or benzylsulphonyl group; a $C_{2-4}$ alkenyl or alkynyl group; or an aryl or aralkyl group containing 6 to 11 carbon atoms and 1 or 2 carbon atoms in any alkyl portion, optionally ring substituted by one or more fluorine, chlorine and/or bromine atoms or by a $C_{1-2}$ alkyl or alkoxy group optionally substituted by one or more fluorine and/or chlorine atoms.

13. A compound as claimed in claim 12, in which $R_3$ represents a hydrogen atom or a $C_{1-3}$ alkyl group optionally substituted by one or more halogen atoms.

14. A compound as claimed in any one of claims 1 to 13, in which both Q's represent hydrogen atoms.

15. A compound as claimed in claim 1, in which $R_3$ is a hydrogen atom or a $C_{1-10}$ alkyl group or when n is 0, then $R_3$ may additionally be a cyano group, a $C_{1-6}$ alkyl group substituted by a hydroxy group, a cyano group, a $C_{1-6}$ alkylsulphonyl group, a phenylsulphonyl group, a benzylsulphonyl group, an azido group, a ($C_{1-6}$ alkoxy)carbonyl group, a hydroxycarbonyl group, a phosphoryl group, a phosphoryloxy group, or an amine oxide, carbamoyl or thiocarbamoyl group in which each nitrogen is optionally substituted by 1 or 2 $C_{1-4}$ alkyl groups; or $R_3$ is a $C_{2-4}$ alkenyl or alkynyl group; an aryl or aralkyl group, each containing from 6 to 11 carbon atoms including 1 to 4 carbon atoms in any alkyl portion and optionally ring substituted by one or more substituents selected from fluorine, chlorine and/or bromine atoms, or by a $C_{1-2}$ alkyl or alkoxy group, each optionally substituted by one or more fluorine and/chlorine atoms; or $R_3$ is a group $—CO_2R_8$ or $—CON(R_8)_2$ in which $R_8$ is a hydrogen atom or a $C_{1-6}$ alkyl group;

each $R_4$ is independently a hydroen atom or a $C_{1-6}$ alkyl group optionally substituted by up to 3 halogen atoms; or one of $R_4$ and $R_1$ together form a carbon-carbon bond; and

W is an alkenyl or alkynyl group containing from 2 to 4 carbon atoms; a 4-pyrimidyl group; a 2-pyrazinyl group; a 3-pyridazinyl group; a 2-pyridyl group; a 2-furyl group; or a phenyl group optionally substituted by one or more of halogen, cyano, amino, $C_{1-3}$ alkoxy or alkylthio each optionally substituted by one or more fluorine and/or chlorine atoms, or $C_{1-2}$ alkyl optionally substituted by one or more fluorine and/or chlorine atoms, hydroxy, $C_{1-2}$ alkoxy, or $C_{1-2}$ alkylthio.

16. A process for the preparation of a compound as claimed in claim 1, which comprises reacting a compound of the general formula

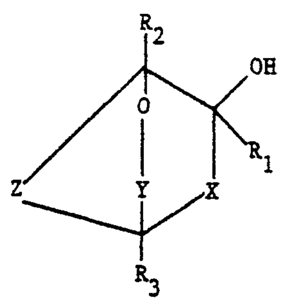

(II)

in which $R_1$, $R_2$, $R_3$, X, Y and Z have the meanings given for the general formula I, with a compound of the general formula $WCQ_2L$, in which W and Q have the meanings given for the general formula I, and L represents a suitable leaving group.

17. A process as claimed in claim 16, carried out in the presence of a strong base.

18. A process as claimed in either claim 16 or claim 17, carried out at a temperature in the range of from 0 to 120°C.

19. A compound as claimed in claim 1, whenever prepared by a process as claimed in any one of claims 16 to 18.

20. A herbicidal composition which comprises a compound as claimed in any one of claims 1 to 15 and 19, together with a carrier and/or a surface-active agent.

21. A process for the control of unwanted plants at a locus, which comprises treating the locus with a compound as claimed in any one of claims 1 to 15 and 19 or a composition as claimed in claim 20.

**Claims for the Contracting State: AT**

1. A herbicidal composition which comprises an active ingredient together with a carrier and/or a surface active agent, characterised in that the active ingredient is a compound of the general formula I

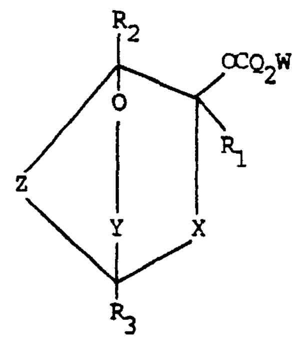

(I)

wherein

X is $—CR_4R_4—$;

Y is $(—CR_5R_6—)_n$ in which n is 0 or 1;

Z is $(—CR_7R_7—)_p$ in which p is 1, 2 or 3;

$R_1$ is a hydrogen atom or $C_{1-6}$ alkyl group optionally substituted by up to 3 fluorine, chlorine and/or bromine atoms;

$R_2$ is a hydrogen atom or a $C_{1-6}$ straight-chain alkyl group;

$R_3$ is a hydrogen atom; a cyano group; a $C_{1-10}$ alkyl group; a $C_{1-6}$ alkyl group which is substituted by one or more halogen atoms or by a hydroxy group, a cyano group, a $C_{1-6}$ alkoxy group, a phenoxy group, a $C_{1-6}$ alkylsulphonyl group, a phenylsulphoryl group, a benzylsulphonyl group, an azido group, a $C_{1-6}$ alkoxycarbonyl group, a benzyloxycarbonyl group, a hydroxycarbonyl group, a phosphoryl group, a phosphoryloxy group, or an amine oxide, carbamoyl or thiocarbamoyl group in which each nitrogen is optionally substituted by 1 or 2 $C_{1-4}$ alkyl groups; a $C_{2-4}$ alkenyl or alkynyl group; an aryl or aralkyl group, each containing from 6 to 11 carbon atoms including 1 to 4 carbon atoms in any alkyl portion, optionally ring substituted by one or more fluorine, chlorine and/or bromine atoms or by a $C_{1-2}$ alkyl or alkoxy group, each optionally substituted by one or more fluorine and/or chlorine atoms; a group $—CSNH_2$; a group $—CO_2R_8$ or $—CON(R_8)_2$ in which $R_8$ is a hydrogen atom or a $C_{1-6}$ alkyl group; or an acetyl group or an oxime or acetal derivative of said group;

each $R_4$ is independently a hydrogen atom; a $C_{1-6}$ alkyl group optionally substituted by up to 3 halogen atoms; a hydroxy group; or a $C_{1-4}$ alkoxy group; or one of $R_4$ and $R_1$ together form a carbon-carbon bond;

$R_5$ and $R_6$ each indendently is a hydrogen atom or a $C_{1-2}$ alkyl group; or when located on a carbon atom adjacent to the ring oxygen atom then $R_5$ and $R_6$ may together form an alkylene group containing 4 or 5 carbon atoms;

each $R_7$ independently is a hydrogen atom or a $C_{1-4}$ alkyl group optionally substituted by up to 3 halogen atoms; or when n is 0 then $R_7$ may also be a chlorine or bromine atom, or two of $R_7$ when located on adjacent carbon atoms together form an epoxide ring or a carbon-carbon bond; or when n is 1, then one $R_7$ on the carbon adjacent to the carbon bearing $R_2$ is a hydroxy group, a $C_{7-11}$ aralkoxy group, or a $C_{1-4}$ alkoxy group, and the other $R_7$ is a hydrogen atom;

both of Q are hydrogen atoms or fluorine atoms; and

W represents a cyano group; a $C_{2-4}$ alkenyl or alkynyl group; a pyrimidinyl, pyrazinyl, pyridazinyl, pyridyl, furyl, naphthyl, imidazolyl, triazolyl, thiadiazolyl, 2-quinolinyl, 1-isoquinolinyl, pyrrolyl, N-methylimidazolyl, N-methylpyrazolyl, isoxazolyl, oxazolyl, isothiazolyl, thiazolyl, thienyl or 5-methyl-2-furyl group; or a cyclohexenyl group, or a cycloalkyl group having up to 6 carbon atoms, optionally substituted by a $C_{1-3}$ alkyl group; or a $C_{3-10}$ secondary alkyl group; or a phenyl group which is unsubstituted or substituted by one or more of the following moieties: hydroxy, cyano, nitro, halogen, $C_{1-3}$ alkoxy, haloalkoxy, alkylthio, haloalkylthio, alkylsulphinyl, haloalkylsulphinyl or alkylsulphonyl; alkenyl or alkynyl of up to 4 carbon atoms; phenyl, phenoxy, benzyl or benzyloxy; amino, aminocarbonyl and carboxyl in each of which hydrogen may be replaced by $C_{1-4}$ alkyl; and $C_{1-3}$ alkyl which is itself optionally substituted by one or more moieties independently selected from halogen, hydroxy, $C_{1-4}$ alkylthio, $C_{1-4}$ alkoxy, and amino.

2. A composition as claimed in claim 1, in which each $R_4$ independently represents a hydrogen atom or a methyl or ethyl group.

3. A composition as claimed in either claim 1 or claim 2, in which each of $R_5$ and $R_6$ independently represents a hydrogen atom or a methyl or ethyl group.

4. A compound as claimed in any one of claims 1 to 3, in which each $R_7$ independently represents a hydrogen atom or a methyl or ethyl group, or when n is 0, a chlorine or bromine atom, or when n is 1, one $R_7$ on the carbon atom adjacent to the carbon atom bearing $R_2$ is a hydroxy, methoxy, ethoxy or benzyloxy group, the or each other $R_7$ being a hydrogen atom.

5. A composition as claimed in claim 4, in which each $R_7$ represents a hydrogen atom.

6. A composition as claimed in any one of claims 1 to 5, in which n is 0 and p is 2.

7. A composition as claimed in any one of claims 1 to 5, in which n is 1 and p is 2.

8. A composition as claimed in any one of claims 1 to 7, in which W represents a $C_{2-4}$ alkenyl or alkynyl group; a 4-pyrimidinyl, 2-pyrazinyl, 3-pyridazinyl, 2-pyridyl or 2-furyl group; or a phenyl group optionally substituted by one or more of halogen, cyano, amino, $C_{1-3}$ alkoxy or alkylthio each optionally substituted by one or more fluorine and/or chlorine atoms, or $C_{1-2}$ alkyl optionally substituted by one or more fluorine and/or chlorine atoms or by hydroxy, $C_{1-2}$ alkoxy or $C_{1-2}$ alkylthio.

9. A composition as claimed in claim 8, in which W represents an ethynyl group; a 2-pyridyl group; or a phenyl group which is unsubstituted or substituted by one or two substituents selected from methyl groups, fluorine atoms and chlorine atoms.

10. A compound as claimed in any one of claims 1 to 9, in which $R_1$ represents a methyl group or a hydrogen atom.

11. A compound as claimed in any one of claims 1 to 10, in which $R_2$ represents a methyl or ethyl group.

12. A compound as claimed in any one of claims 1 to 11, in which $R_3$ represents a hydrogen atom; a $C_{1-6}$ alkyl group optionally substituted by up to 3 fluorine, chlorine and/or bromine atoms or by a hydroxy, cyano, $C_{1-6}$ alkoxy, $C_{1-6}$ alkylsulphonyl, phenylsulphonyl or benzylsulphonyl group; a $C_{2-4}$ alkenyl or alkynyl group; or an aryl or aralkyl group containing 6 to 11 carbon atoms and 1 or 2 carbon atoms in any alkyl portion, optionally ring substituted by one or more fluorine, chlorine and/or bromine atoms or by a $C_{1-2}$ alkyl or alkoxy group optionally substituted by one or more fluorine and/or chlorine atoms.

13. A composition as claimed in claim 12, in which $R_3$ represents a hydrogen atom or a $C_{1-3}$ alkyl group optionally substituted by one or more halogen atoms.

14. A composition as claimed in any one of claims 1 to 13, in which both Q's represent hydrogen atoms.

15. A composition as claimed in claim 1, in which $R_3$ is a hydrogen atom or a $C_{1-10}$ alkyl group or when n is 0, then $R_3$ may additionally be a cyano group, a $C_{1-6}$ alkyl group substituted by a hydroxy group, a cyano group, a $C_{1-6}$ alkylsulphonyl group, a phenylsulphonyl group, a benzylsulphonyl group, an azido group, a ($C_{1-6}$ alkoxy)carbonyl group, a hydroxycarbonyl group, a phosphoryl group, a phosphoryloxy group, or an amine oxide, carbamoyl or thiocarbamoyl group in which each nitrogen is optionally substituted by 1 or 2 $C_{1-4}$ alkyl groups; or $R_3$ is a $C_{2-4}$ alkenyl or alkynyl group; an aryl or aralkyl group, each containing from 6 to 11 carbon atoms including 1 to 4 carbon atoms in any alkyl portion and optionally ring substituted by one or more substituents selected from fluorine, chlorine and/or bromine atoms, or by a $C_{1-2}$ alkyl or alkoxy group, each optionally substituted by one or more fluorine and/chlorine atoms; or $R_3$ is a group $-CO_2R_8$ or $-CON(R_8)_2$ in which $R_8$ is a hydrogen atom or a $C_{1-6}$ alkyl group;

each $R_4$ is independently a hydrogen atom or a $C_{1-6}$ alkyl group optionally substituted by up to 3 halogen atoms; or one of $R_4$ and $R_1$ together form a carbon-carbon bond; and

W is an alkenyl or alkynyl group containing from 2 to 4 carbon atoms; a 4-pyrimidyl group; a 2-pyrazinyl group; a 3-pyridazinyl group; a 2-pyridyl group; a 2-furyl group; or a phenyl group optionally substituted by one or more of halogen, cyano, amino, $C_{1-3}$ alkoxy or alkylthio each optionally substituted by one or more fluorine and/or chlorine atoms, or $C_{1-2}$ alkyl optionally substituted by one or more fluorine and/or chlorine atoms, hydroxy, $C_{1-2}$ alkoxy, or $C_{1-2}$ alkylthio.

16. A process for the preparation of a compound as claimed in claim 1, which comprises reacting a compound of the general formula

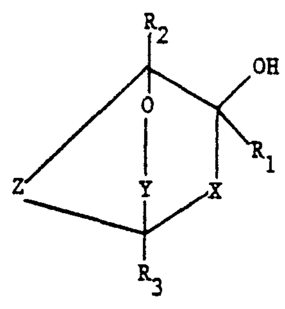

(II)

in which $R_1$, $R_2$, $R_3$, X, Y and Z have the meanings given for the general formula I, with a compound of the general formula $WCQ_2L$, in which W and Q have the meanings given for the general formula I, and L represents a suitable leaving group.

17. A process as claimed in claim 16, carried out in the presence of a strong base.

18. A process as claimed in either claim 16 or claim 17, carried out at a temperature in the range of from 0 to 120°C.

19. A process for the control of unwanted plants at a locus, which comprises treating the locus with a composition as claimed in any one of claims 1 to 15.

**Patentansprüche für die Vertragsstaaten: BE CH DE FR GB IT LI LU NL SE**

1. Verbindung der allgemeinen Formel I

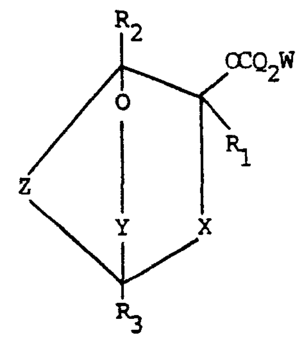

(I)

worin

X für $-CR_4R_4-$ steht;

Y für $(-CR_5R_6-)_n$ steht, worin n den Wert 0 oder 1 aufweist;

Z für $(-CR_7R_7-)_p$ steht, worin p den Wert 1, 2 oder 3 aufweist;

$R_1$ ein Wasserstoffatom oder eine $C_{1-6}$-Alkylgruppe bedeutet, die gegebenenfalls durch bis zu 3 Fluor-, Chlor- und/oder Bromatome substituiert ist;

$R_2$ ein Wasserstoffatom oder eine $C_{1-6}$-geradkettige Alkylgruppe bedeutet;

$R_3$ ein Wasserstoffatom; eine Cyanogruppe; eine $C_{1-10}$-Alkylgruppe; eine $C_{1-6}$-Alkylgruppe, die durch ein oder mehrere Halogenatome oder durch eine Hydroxygruppe, eine Cyanogruppe, eine $C_{1-6}$-Alkoxygruppe, eine Phenoxygruppe, eine $C_{1-6}$-Alkylsulfonylgruppe, eine Phenylsulfonylgruppe, eine Benzylsulfonylgruppe, eine Azidogruppe, eine $C_{1-6}$-Alkoxycarbonylgruppe, eine Benzyloxycarbonylgruppe, eine Hydroxycarbonylgruppe, eine Phosphorylgruppe, eine Phosphoryloxygruppe oder eine Aminoxid-, Carbamoyl- oder Thiocarbamoylgruppe substituiert ist, worin jeder Stickstoff gewünschtenfalls durch eine oder zwei $C_{1-4}$-Alkylgruppen substituiert ist; eine $C_{2-4}$-Alkenyl- oder -Alkinylgruppe; eine Aryl- oder Aralkylgruppe, jeweils enthaltend von 6 bis 11 Kohlenstoffatomen einschließlich 1 bis 4 Kohlenstoffatomen in einem etwa vorliegenden Alkylteil, gegebenenfalls im Ring substituiert durch ein oder mehrere Fluor-, Chlor- und/oder Bromatome oder durch eine $C_{1-2}$-Alkyl- oder Alkoxygruppe, die jeweils im gegebenen Falle durch ein oder mehrere Fluor- und/oder Chloratome substituiert sind; eine Gruppe $-CSNH_2$; eine Gruppe $-CO_2R_8$ oder $-CON(R_8)_2$, worin $R_8$ ein Wasserstoffatom oder eine $C_{1-6}$-Alkylgruppe bedeutet; oder eine Acetylgruppe oder ein Oxim oder Acetalderivat dieser Gruppe bedeutet;

jeder Rest $R_4$ unabhängig ein Wasserstoffatom; eine $C_{1-6}$-Alkylgruppe, die gegebenenfalls durch bis zu 3 Halogenatome substituiert ist; eine Hydroxygruppe; oder eine $C_{1-4}$-Alkoxygruppe bedeutet; oder ein Rest $R_4$ und $R_1$ zusammen eine Kohlenstoff-Kohlenstoff-Bindung ausbilden;

$R_5$ und $R_6$ jeweils unabhängig ein Wasserstoffatom oder eine $C_{1-2}$-Alkylgruppe bedeuten, oder dann, wenn $R_5$ und $R_6$ an einem dem Ringsauerstoff benachbarten Kohlenstoffatom angeordnet sind, gemeinsam eine 4 oder 5 Kohlenstoffatome enthaltende Alkylengruppe ausbilden können;

jeder Rest $R_7$ unabhängig ein Wasserstoffatom oder eine $C_{1-4}$-Alkylgruppe bedeutet, die gegebenenfalls durch bis zu 3 Halogenatome substituiert ist; oder, falls n 0 ist, dann $R_7$ such ein Chlor- oder Bromatom sein kann, oder zwei Reste $R_7$, wenn sie an benachbarten Kohlenstoffatomen angeordnet sind, zusammen einen Epoxidring oder eine Kohlenstoff-Kohlenstoff-Bindung ausbilden können; oder,

wenn n den Wert 1 hat, dann ein Rest $R_7$ an dem Kohlenstoffatom, der dem den Rest $R_2$ tragenden Kohlenstoffatom benachbart ist, eine Hydroxygruppe, eine $C_{7-11}$-Aralkoxygruppe oder eine $C_{1-4}$-Alkoxygruppe ist und der andere Rest $R_7$ ein Wasserstoffatom ist;

beide Rest Q Wasserstoffatome oder Fluoratome bedeuten; und

W eine Cyanogruppe; eine $C_{2-4}$-Alkenyl- oder -Alkinylgruppe; eine Pyrimidinyl-, Pyrazinyl-, Pyridazinyl-, Pyridyl-, Furyl-, Naphthyl-, Imidazolyl-, Triazolyl-, Thiadiazolyl-, 2-Chinolinyl-, 1-Isochinolinyl-, Pyrrolyl-, N-Methylimidazolyl-, N-Methylpyrazolyl-, Isoxazolyl-, Oxazolyl-, Isothiazolyl-, Thiazolyl-, Thienyl- oder 5-Methyl-2-furylgruppe; oder eine Cyclohexenylgruppe, oder eine Cycloalkylgruppe mit bis zu 6 Kohlenstoffatomen, gegebenenfalls substituiert durch eine $C_{1-3}$-Alkylgruppe; oder eine $C_{3-10}$sek.Alkylgruppe; oder eine Phenylgruppe bedeutet, die unsubstituiert oder durch einen oder mehrere der folgenden Rest substituiert ist; Hydroxy, Cyano, Nitro, halogen, $C_{1-3}$-Alkoxy, Halogenalkoxy, Alkylthio, Halogenalkylthio, Alkylsulfinyl, Halogenalkylsulfinyl oder Alkylsulfonyl; Alkenyl oder Alkinyl mit bis zu 4 Kohlenstoffatomen; Phenyl, Phenoxy, Benzyl oder Benzyloxy; Amino, Aminocarbonyl und Carboxyl, in welchen jeweils Wasserstoff durch $C_{1-4}$ -Alkyl ersetzt sein kann; und $C_{1-3}$-Alkyl, das seinerseits gegebenenfalls durch einen oder mehrere Reste, unabhängig ausgewählt unter Halogen, Hydroxy, $C_{1-4}$-Alkylthio, $C_{1-4}$-Alkoxy und Amino, substituiert ist.

2. Verbindung nach Anspruch 1, worin jeder Rest $R_4$ unabhängig ein Wasserstoffatom oder eine Methyl- oder Ethylgruppe darstellt.

3. Verbindung nach Anspruch 1 oder Anspruch 2, worin jeder der Reste $R_5$ und $R_6$ unabhängig ein Wasserstoffatom oder eine Methyl- oder Ethylgruppe bedeutet.

4. Verbindung nach einem der Ansprüche 1 bis 3, worin jeder Rest $R_7$ unabhängig ein Wasserstoffatom oder eine Methyl- oder Ethylgruppe oder, falls n den Wert 0 hat, ein Chlor- oder Bromatom darstellt, oder, falls n den Wert 1 hat, der eine Rest $R_7$ an dem Kohlenstoffatom, das dem den Rest $R_2$ tragenden Kohlenstoffatom benachbart ist, eine Hydroxy-, Methoxy-, Ethoxy- oder Benzyloxygruppe darstellt, während der oder jede weitere Rest $R_7$ ein Wasserstoffatom bedeutet.

5. Verbindung nach Anspruch 4, worin jeder Rest $R_7$ ein Wasserstoffatom darstellt.

6. Verbindung nach einem der Ansprüche 1 bis 5, worin n den Wert 0 hat und p den Wert 2 aufweist.

7. Verbindung nach einem der Ansprüche 1 bis 5, worin n den Wert 1 hat und p den Wert 2 aufweist.

8. Verbindung nach einem der Ansprüche 1 bis 7, worin W eine $C_{2-4}$-Alkenyl- oder -Alkinylgruppe; eine 4-Pyrimidinylgruppe; eine 2-Pyrazinylgruppe; eine 3-Pyrazinylgruppe; eine 2-Pyridylgruppe; eine 2-Furylgruppe; oder eine Phenylgruppe bedeutet, die gegebenenfalls durch einen oder mehrere der Reste Halogen, Cyano, Amino, $C_{1-3}$-Alkoxy oder Alkylthio, jeweils gegebenefalls substituiert durch ein oder mehrere Fluor- und/oder Chloratome, oder $C_{1-2}$-Alkyl substituiert ist, das gegebenenfalls durch ein oder mehrere Fluor- und/oder Chloratome oder durch Hydroxy, $C_{1-2}$-Alkoxy oder $C_{1-2}$-Alkylthio substituiert ist.

9. Verbindung nach Anspruch 8, worin W eine Ethinylgruppe; eine 2-Pyridylgruppe; oder eine Phenylgruppe bedeutet, die unsubstituiert oder durch einen oder zwei Substituenten, ausgewählt unter Methylgruppen, Fluoratomen und Chloratomen, substituiert ist.

10. Verbindung nach einem der Ansprüche 1 bis 9, worin $R_1$ eine Methylgruppe oder ein wasserstoffatom darstellt.

11. Verbindung nach einem der Ansprüche 1 bis 10, worin $R_2$ eine Methyl- oder Ethylgruppe darstellt.

12. Verbindung nach einem der Ansprüche 1 bis 11, worin $R_3$ ein Wasserstoffatom; ein $C_{1-6}$-Alkylgruppe, die gegebenenfalls durch bis zu 3-Fluor-, Chlor- und/oder Bromatome oder durch eine Hydroxy-, Cyano-, $C_{1-6}$-Alkoxy-, $C_{1-6}$-Alkylsulfonyl-, Phenylsulfonyl- oder Benzylsulfonylgruppe substituiert ist; eine $C_{2-4}$-Alkenyl- oder -Alkinylgruppe; oder eine Aryl- oder Aralkylgruppe mit 6 bis 11 Kohlenstoffatomen und 1 oder 2 Kohlenstoffatomen in einem etwa vorliegenden Alkylteil, die gegebenenfalls im Ring durch ein oder mehrere Fluor-, Chlor- und/oder Bromatome oder durch eine $C_{1-2}$-Alkyl- oder -Alkoxygruppe substituiert ist, die gegebenenfalls durch ein oder mehrere Fluor- und/oder Chloratome substituiert ist, darstellt.

13. Verbindung nach Anspruch 12, worin $R_3$ ein Wasserstoffatom oder eine $C_{1-3}$-Alkylgruppe darstellt, die gegebenenfalls durch ein oder mehrere Halogenatome substituiert ist.

14. Verbindung nach einem der Ansprüche 1 bis 13, worin beide Reste Q Wasserstoffatome bedeuten.

15. Verbindung nach Anspruch 1, worin $R_3$ ein Wasserstoffatom oder eine $C_{1-10}$-Alkylgruppe darstellt oder, wenn n den Wert 0 hat, dann $R_3$ zusätzlich eine Cyanogruppe oder eine $C_{1-6}$-Alkylgruppe sein kann, die durch eine Hydroxygruppe, eine Cyanogruppe, eine $C_{1-6}$-Alkoxygruppe, eine Aryloxygruppe, eine $C_{1-6}$-Alkylsulfonylgruppe, eine Phenylsulfonylgruppe, eine Benzylsulfonylgruppe, eine Azidogruppe, eine ($C_{1-6}$-Alkoxy)carbonylgruppe, eine Hydroxycarbonylgruppe, eine Phosphorylgruppe, eine Phosphoryloxygruppe oder eine Aminoxid-, Carbamoyl- oder Thiocarbamoylgruppe substituiert ist, worin jeder Stickstoff gegebenenfalls durch 1 oder 2 $C_{1-4}$-Alkylgruppen substituiert ist; oder $R_3$ eine Alkenyl- oder Alkinylgruppe mit 2 bis 4 Kohlenstoffatomen; eine Aryl- oder Aralkylgruppe, jeweils enthaltend 6 bis 11 Kohlenstoffatome einschließlich 1 bis 4 Kohlenstoffatomen in einem etwa vorliegenden Alkylteil und gegebenenfalls ringsubstituiert durch einen oder mehrere Substituenten, ausgewählt unter Fluor-, Chlor- und/oder Bromatomen, oder durch eine $C_{1-2}$-Alkyl- oder Alkoxygruppe, jeweils gegebenenfalls substituiert durch ein oder mehrere Fluor- und/oder Chloratome; oder $R_3$ eine Gruppe $—CO_2R_8$ oder $—CON(R_8)_2$ bedeutet, worin $R_8$ ein Wasserstoffatom oder eine $C_{1-6}$-Alkylgruppe darstellt;

jeder Rest $R_4$ unabhängig ein Wasserstoffatom oder eine $C_{1-6}$-Alkylgruppe bedeutet, die gegebenenfalls durch bis zu 3 Halogenatome substituiert ist; oder einer der Reste $R_4$ und $R_1$ zusammen eine Kohlenstoff-Kohlenstoff-Bindung ausbilden; und

W eine Alkenyl- oder Alkinylgruppe mit 2 bis 4 Kohlenstoffatomen; eine 4-Pyrimidylgruppe; eine 2-Pyrazinylgruppe; eine 3-Pyridazinylgruppe; eine 2-Pyridylgruppe; eine 2-Furylgruppe; oder eine Phenylgruppe bedeutet, die gegenbenenfalls durch ein oder mehrere Halogene, Cyano, Amino, $C_{1-3}$-Alkoxy oder Alkylthio, jeweils gegebenenfalls substituiert durch ein oder mehrere Fluor- und/oder Chloratome, oder $C_{1-2}$-Alkyl, gegebenenfalls substituiert durch ein oder mehrere Fluor- und/oder Chloratome, Hydroxy, $C_{1-2}$-Alkoxy oder $C_{1-2}$-Alkylthio, substituiert ist.

16. Verfahren zur Herstellung einer Verbindung, wie in Anspruch 1 beansprucht, welches ein Umsetzen einer Verbindung der allgemeinen Formel

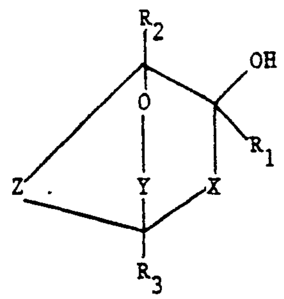

(II)

worin $R_1$, $R_2$, $R_3$, X, Y und Z die für die allgemeinen Formel I angegebenen Bedeutungen besitzen, mit einer Verbindung der allgemeinen Formel $WCQ_2L$, worin W und Q die für die allgemeine Formel I angegebenen Bedeutungen besitzen und L eine geeignete Leaving-Gruppe darstellt, umfaßt.

17. Verfahren nach Anspruch 16, welches in Gegenwart einer starken Base ausgeführt wird.

18. Verfahren nach Anspruch 16 oder Anspruch 17, welches bei einer Temperatur im Bereich von 0 bis 120°C ausgeführt wird.

19. Verbindung nach Anspruch 1, wenn sie nach einem Verfahren gemäß einem der Ansprüche 16 bis 18 hergestellt ist.

20. Herbicide Zusammensetzung, welche eine Verbindung, wie in einem der Ansprüche 1 bis 15 und 19 beansprucht, zusammen mit einem Träger und/oder einem oberflächenaktiven Mittel umfaßt.

21. Verfahren zur Bekämpfung unerwünschter Pflanzen an einem Ort, welches ein Behandeln des Ortes mit einer Verbindung, wie in einem der Ansprüche 1 bis 15 und 19 beansprucht, oder mit einer Zusammensetzung, wie in Anspruch 20 beansprucht, umfaßt.

**Patentansprüche für den Vertragsstaat: AT**

1. Herbicide Zusammensetzung, welche einen wirksamen Bestandteil zusammen mit einem Träger und/oder einem ober- flachenaktiven Mittel umfaßt, dadurch gekennzeichnet, daß der wirksame Bestandteil eine Verbindung der allgemeinen Formel I

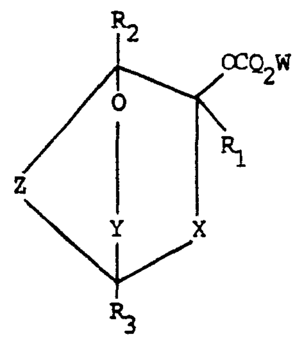

(I)

ist, worin

X für $—CR_4R_4—$ steht;

Y für $(—CR_5R_6—)_n$ steht, worin n den Wert 0 oder 1 aufweist;

Z für $(—CR_7R_7—)_p$ steht, worin p den Wert 1, 2 oder 3 aufweist;

$R_1$ ein Wasserstoffatom oder eine $C_{1-6}$-Alkylgruppe bedeutet, die gegebenenfalls durch bis zu 3 Fluor-, Chlor- und/oder Bromatome substituiert ist;

$R_2$ ein Wasserstoffatom oder eine $C_{1-6}$-geradkettige Alkylgruppe bedeutet;

$R_3$ ein Wasserstoffatom; eine Cyanogruppe; eine $C_{1-10}$-Alkylgruppe; eine $C_{1-6}$-Alkylgruppe, die durch ein oder mehrere Halogenatome oder durch eine Hydroxygruppe, eine Cyanogruppe, eine $C_{1-6}$-Alkoxygruppe, eine Phenoxygruppe, eine $C_{1-6}$-Alkylsulfonylgruppe, eine Phenylsulfonylgruppe, eine Benzylsulfonylgruppe, eine Azidogruppe, eine $C_{1-6}$-Alkoxycarbonylgruppe, eine Benzyloxycarbonylgruppe, eine Hydroxycarbonylgruppe, eine Phosphorylgruppe, eine Phosphoryloxygruppe oder eine Aminoxid-, Carbamoyl- oder Thiocarbamoylgruppe substituiert ist, worin jeder Stickstoff gewünschtenfalls durch eine oder zwei $C_{1-4}$-Alkylgruppen substituiert ist; eine $C_{2-4}$-Alkenyl- oder -Alkinylgruppe; eine Aryl- oder Aralkylgruppe, jeweils enthaltend von 6 bis 11 Kohlenstoffatomen einschließlich 1 bis 4 Kohlenstoffatomen in einem etwa vorliegenden Alkylteil, gegebenenfalls im Ring substituiert durch ein oder mehrere Fluor-, Chlor- und/oder Bromatome oder durch eine $C_{1-2}$-Alkyl- oder Alkoxygruppe, die jeweils im gegebenen Falle durch ein oder mehrere Fluor- und/oder Chloratome substituiert sind; eine Gruppe $—CSNH_2$; eine Gruppe $—CO_2R_8$ oder $—CON(R_8)_2$, worin $R_8$ ein

Wasserstoffatom oder eine $C_{1-6}$-Alkylgruppe bedeutet; oder eine Acetylgruppe oder ein Oxim oder Acetalderivat dieser Gruppe bedeutet;

jeder Rest $R_4$ unabhängig ein Wasserstoffatom; eine $C_{1-6}$-Alkylgruppe, die gegebenenfalls durch bis zu 3 Halogenatome substituiert ist; eine Hydroxygruppe; oder eine $C_{1-4}$-Alkoxygruppe bedeutet; oder ein Rest $R_4$ und $R_1$ zusammen eine Kohlenstoff-Kohlenstoff-Bindung ausbilden;

$R_5$ und $R_6$ jeweils unabhängig ein Wasserstoffatom oder eine $C_{1-2}$-Alkylgruppe bedeuten, oder dann, wenn $R_5$ und $R_6$ an einem dem Ringsauerstoff benachbarten Kohlenstoffatom angeordnet sind, gemeinsam eine 4 oder 5 Kohlenstoffatome enthaltende Alkylengruppe ausbilden können;

jeder Rest $R_7$ unabhängig ein Wasserstoffatom oder eine $C_{1-4}$-Alkylgruppe bedeutet, die gegebenenfalls durch bis zu 3 Halogenatome substituiert ist; oder, falls n 0 ist, dann $R_7$ auch ein Chlor- oder Bromatom sein kann, oder zwei Reste $R_7$, wenn sie an benachbarten Kohlenstoffatomen angeordnet sind, zusammen einen Epoxidring oder eine Kohlenstoff-Kohlenstoff-Bindung ausbilden können; oder,

wenn n den Wert 1 hat, dann ein Rest $R_7$ an dem Kohlenstoffatom, der dem den Rest $R_2$ tragenden Kohlenstoffatom benachbart ist, eine Hydroxygruppe, eine $C_{7-11}$-Aralkoxygruppe oder eine $C_{1-4}$-Alkoxygruppe ist und der andere Rest $R_7$ ein Wasserstoffatom ist;

beide Rest Q Wasserstoffatome oder Fluoratome bedeuten; und

W eine Cyanogruppe; eine $C_{2-4}$-Alkenyl- oder -Alkinylgruppe; eine Pyrimidinyl-, Pyrazinyl-, Pyridazinyl-, Pyridyl-, Furyl-, Naphthyl-, Imidazolyl-, Triazolyl-, Thiadiazolyl-, 2-Chinolinyl-, 1-Isochinolinyl-, Pyrrolyl-, N-Methylimidazolyl-, N-Methylpyrazolyl-, Isoxazolyl-, Oxazolyl-, Isothiazolyl-, Thiazolyl-, Thienyl- oder 5-Methyl-2-furylgruppe; oder eine Cyclohexenylgruppe, oder eine Cycloalkylgruppe mit bis zu 6 Kohlenstoffatomen, gegebenenfalls substituiert durch eine $C_{1-3}$-Alkylgruppe; oder eine $C_{3-10}$sek.Alkylgruppe; oder eine Phenylgruppe bedeutet, die unsubstituiert oder durch einen oder mehrere der folgenden Reste substituiert ist: Hydroxy, Cyano, Nitro, Halogen, $C_{1-3}$-Alkoxy, Halogenalkoxy, Alkylthio, Halogenalkylthio, Alkylsulfinyl, Halogenalkylsulfinyl oder Alkylsulfonyl; Alkenyl oder Alkinyl mit bis zu 4 Kohlenstoffatomen; Phenyl, Phenoxy, Benzyl oder Benzyloxy; Amino, Aminocarbonyl und Carboxyl, in welchen jeweils Wasserstoff durch $C_{1-4}$-Alkyl ersetzt sein kann; und $C_{1-3}$-Alkyl, das seinerseits gegebenenfalls durch einen oder mehrere Reste, unabhängig ausgewählt unter Halogen, Hydroxy, $C_{1-4}$-Alkylthio, $C_{1-4}$-Alkoxy und Amino, substituiert ist.

2. Zusammensetzung nach Anspruch 1, worin jeder Rest $R_4$ unabhängig ein Wasserstoffatom oder eine Methyl- oder Ethylgruppe darstellt.

3. Zusammensetzung nach Anspruch 1 oder Anspruch 2, worin jeder der Reste $R_5$ und $R_6$ unabhängig ein Wasserstoffatom oder eine Methyl- oder Ethylgruppe bedeutet.

4. Zusammensetzung nach einem der Ansprüche 1 bis 3, worin jeder Rest $R_7$ unabhängig ein Wasserstoffatom oder eine Methyl- oder Ethylgruppe oder, falls n den Wert 0 hat, ein Chlor- oder Bromatom darstellt, oder, falls n den Wert 1 hat, der eine Rest $R_7$ an dem Kohlenstoffatom, das dem den Rest $R_2$ tragenden Kohlenstoffatom benachbart ist, eine Hydroxy-, Methoxy-, Ethoxy- oder Benzyloxygruppe darstellt, während der oder jede weitere Rest $R_7$ ein Wasserstoffatom bedeutet.

5. Zusammensetzung nach Anspruch 4, worin jeder Rest $R_7$ ein Wasserstoffatom darstellt.

6. Zusammensetzung nach einem der Ansprüche 1 bis 5, worin n den Wert 0 hat und p den Wert 2 aufweist.

7. Zusammensetzung nach einem der Ansprüche 1 bis 5, worin n den Wert 1 hat und p den Wert 2 aufweist.

8. Zusammensetzung nach einem der Ansprüche 1 bis 7, worin W eine $C_{2-4}$-Alkenyl- oder -Alkinylgruppe; eine 4-Pyrimidinylgruppe; eine 2-Pyrazinylgruppe; eine 3-Pyrazinylgruppe; eine 2-Pyridylgruppe; eine 2-Furylgruppe; oder eine Phenylgruppe bedeutet, die gegebenenfalls durch einen oder mehrere der Reste Halogen, Cyano, Amino, $C_{1-3}$-Alkoxy oder Alkylthio, jeweils gegebenenfalls substituiert durch ein oder mehrere Fluor- und/oder Chloratome, oder $C_{1-2}$-Alkyl substituiert ist, das gegebenenfalls durch ein oder mehrere Fluor- und/oder Chloratome oder durch Hydroxy, $C_{1-2}$-Alkoxy oder $C_{1-2}$-Alkylthio substituiert ist.

9. Zusammensetzung nach Anspruch 8, worin W eine Ethinylgruppe; eine 2-Pyridylgruppe; oder eine Phenylgruppe bedeutet, die unsubstituiert oder durch einen ode zwei Substituenten, ausgewählt unter Methylgruppen, Fluoratomen und Chloratomen, substituiert ist.

10. Zusammensetzung nach einem der Ansprüche 1 bis 9, worin $R_1$ eine Methylgruppe oder ein Wasserstoffatom darstellt.

11. Zusammensetzung nach einem der Ansprüche 1 bis 10, worin $R_2$ eine Methyl- oder Ethylgruppe darstellt.

12. Zusammensetzung nach einem der Ansprüche 1 bis 11, worin $R_3$ ein Wasserstoffatom; ein $C_{1-6}$-Alkylgruppe, die gegebenenfalls durch biz zu 3-Fluor-, Chlor- und/oder Bromatome oder durch eine Hydroxy-, Cyano-, $C_{1-6}$-Alkoxy-, $C_{1-6}$-Alkylsulfonyl-, Phenylsulfonyl- oder Benzylsulfonylgruppe substituiert ist; eine $C_{2-4}$-Alkenyl- oder -Alkinylgruppe; oder eine Aryl- oder Aralkylgruppe mit 6 bis 11 Kohlenstoffatomen und 1 oder 2 Kohlenstoffatomen in einem etwa vorliegenden Alkylteil, die gegebenenfalls im Ring durch ein oder mehrere Fluor-, Chlor- und/oder Bromatome oder durch eine $C_{1-2}$-Alkyl- oder -Alkoxygruppe substituiert ist, die gegebenenfalls durch ein oder mehrere Fluor- und/oder Chloratome substituiert ist, darstellt.

13. Zusammensetzung nach Anspruch 12, worin $R_3$ ein Wasserstoffatom oder eine $C_{1-3}$-Alkylgruppe

darstellt, die gegebenenfalls durch ein oder mehrere Halogenatome substituiert ist.

14. Zusammensetzung nach einem der Ansprüche 1 bis 13, worin beide Reste Q Wasserstoffatome bedeuten.

15. Zusammensetzung nach Anspruch 1, worin $R_3$ ein Wasserstoffatom oder eine $C_{1-10}$-Alkylgruppe darstellt oder, wenn n den Wert 0 hat, dann $R_3$ zusätzlich eine Cyanogruppe oder eine $C_{1-6}$-Alkylgruppe sein kann, die durch eine Hydroxygruppe, eine Cyanogruppe, eine $C_{1-6}$-Alkoxygruppe, eine Aryloxygruppe, eine $C_{1-6}$-Alkylsulfonylgruppe, eine Phenylsulfonylgruppe, eine Benzylsulfonylgruppe, eine Azidogruppe, eine ($C_{1-6}$-Alkoxy)carbonylgruppe, eine Hydroxycarbonylgruppe, eine Phosphorylgruppe, eine Phosphoryloxygruppe oder eine Aminoxid-, Carbamoyl- oder Thiocarbamoylgruppe substituiert ist, worin jeder Stickstoff gegebenenfalls durch 1 oder 2 $C_{1-4}$-Alkylgruppen substituiert ist; oder $R_3$ eine Alkenyl- oder Alkinylgruppe mit 2 bis 4 Kohlenstoffatomen; eine Aryl- oder Aralkylgruppe, jeweils enthaltend 6 bis 11 Kohlenstoffatome einschließlich 1 bis 4 Kohlenstoffatomen in einem etwa vorliegenden Alkylteil und gegebenenfalls ringsubstituiert durch einen oder mehrere Substituenten, ausgewählt unter Fluor-, Chlor- und/oder Bromatomen, oder durch eine $C_{1-2}$-Alkyl- oder Alkoxygruppe, jeweils gegebenenfalls substituiert durch ein oder mehrere Fluor- und/oder Chloratome; oder $R_3$ eine Gruppe $-CO_2R_8$ oder $-CON(R_8)_2$ bedeutet, worin $R_8$ ein Wasserstoffatom oder eine $C_{1-6}$-Alkylgruppe darstellt;

jeder Rest $R_4$ unabhängig ein Wasserstoffatom oder eine $C_{1-6}$-Alkylgruppe bedeutet, die gegebenenfalls durch bis zu 3 Halogenatome substituiert ist; oder einer der Reste $R_4$ und $R_1$ zusammen eine Kohlenstoff-Kohlenstoff-Bindung ausbilden; und

W eine Alkenyl- oder Alkinylgruppe mit 2 bis 4 Kohlenstoffatomen; eine 4-Pyrimidylgruppe; eine 2-Pyrazinylgruppe; eine 3-Pyridazinylgruppe; eine 2-Pyridylgruppe; eine 2-Furylgruppe; oder eine Phenylgruppe bedeutet, die gegenbenenfalls durch ein oder mehrere Halogene, Cyano, Amino, $C_{1-3}$-Alkoxy oder Alkylthio, jeweils gegebenenfalls substituiert durch ein oder mehrere Fluor- und/oder Chloratome, oder $C_{1-2}$-Alkyl, gegebenenfalls substituiert durch ein oder mehrere Fluor- und/oder Chloratome, Hydroxy, $C_{1-2}$-Alkoxy oder $C_{1-2}$-Alkylthio, substituiert ist.

16. Verfahren zur Herstellung einer Verbindung, wie in Anspruch 1 beansprucht, welches ein Umsetzen einer Verbindung der allgemeinen Formel

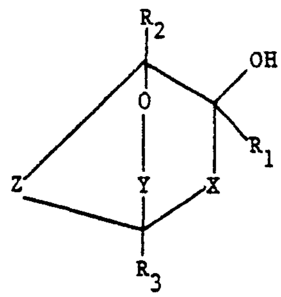

(II)

worin $R_1$, $R_2$, $R_3$, X, Y und Z die für die allgemeinen Formel I angegebenen Bedeutungen besitzen, mit einer Verbindung der allgemeine Formel $WCQ_2L$, worin W und Q die für die allgemeine Formel I angegebenen Bedeutungen besitzen und L eine geeignete Leaving-Gruppe darstellt, umfaßt.

17. Verfahren nach Anspruch 16, welches in Gegenwart einer starken Base ausgeführt wird.

18. Verfahren nach Anspruch 16 oder Anspruch 17, welches bei einer Temperatur im Bereich von 0 bis 120°C ausgeführt wird.

19. Verfahren zur Bekämpfung unerwünschter Pflanzen an einem Ort, welches ein Behandeln des Ortes mit einer Zusammensetzung, wie in einem der Ansprüche 1 bis 15 beansprücht, umfaßt.

**Revendications pour les Etats contractants: BE CH DE FR GB IT LI LU NL SE**

1. Un composé de la formule générale I

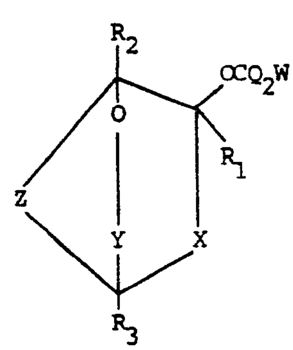

(I)

dans laquelle

X est $-CR_4R_4-$;

Y est $(-CR_5R_6-)_n$ où n est 0 ou 1;

Z est $(-CR_7R_7-)_p$ où p est 1, 2 ou 3;

$R_1$ est un atome d'hydrogène ou un groupe alcoyle en $C_{1-6}$ éventuellement substitué par jusqu'à 3 atomes de fluor, de chlore et/ou de brome;

$R_2$ est un atome d'hydrogène ou un groupe alcoyle en $C_{1-6}$ à chaîne droite;

$R_3$ est un atome d'hydrogène; un groupe cyano; un groupe alcoyle en $C_{1-10}$; un groupe alcoyle en $C_{1-6}$ qui est substitué par un ou plusieurs atomes d'halogènes ou par un groupe hydroxy, un groupe cyano, un groupe alcoxy en $C_{1-6}$, un groupe phénoxy, un groupe $C_{1-6}$ alcoylsulfonyle, un groupe phénylsulfonyle, un groupe benzylsulfonyle, un groupe azido, un groupe $C_{1-6}$ alcoxycarbonyle, un groupe benzyloxycarbonyle, un groupe hydroxycarbonyle, un groupe phosphoryle, un groupe phosphoryloxy ou un groupe oxyde d'amine, carbamoyle ou thiocarbamoyle dans lequel chaque atome d'azote est éventuellement substitué par 1 ou 2 groupes alcoyle en $C_{1-4}$; un groupe alcényle ou alcynyle en $C_{2-4}$; un groupe aryle ou aralcoyle, contenant chacun de 6 à 11 atomes de carbone comprenant 1 à 4 atomes de carbone dans toute portion alcoyle, éventuellement substitué au noyau par un ou plusieurs atomes de fluor, de chlore, et/ou de brome ou par un groupe alcoyle en $C_{1-2}$ ou alcoxy, chacun éventuellement substitué par un ou plusieurs atomes de fluor et/ou de chlore; un groupe $-CSNH_2$; un groupe $-CO_2R_8$ ou $-CON(R_8)_2$ dans lequel $R_8$ est un atome d'hydrogène ou un groupe alcoyle en $C_{1-6}$; ou un groupe acétyle ou un dérivé oxime ou acétal de ce groupe;

chaque $R_4$ est indépendamment un atome d'hydrogène; un groupe alcoyle en $C_{1-6}$ éventuellement substitué par jusqu'à 3 atomes d'halogènes; un groupe hydroxy; ou un groupe alcoxy en $C_{1-4}$; ou un des $R_4$ et $R_1$ forment ensemble une liaison carbone-carbone;

$R_5$ et $R_6$ sont chacun indépendamment un atome d'hydrogène ou un groupe alcoyle en $C_{1-2}$; ou quand ils sont situés sur un atome de carbone adjacent à l'atome d'oxygène du noyay, alors $R_5$ et $R_6$ peuvent former ensemble un groupe alcoylène contenant 4 ou 5 atomes de carbone;

chaque $R_7$ indépendamment est un atome d'hydrogène ou un groupe alcoyle en $C_{1-4}$ éventuellement substitué par jusqu'à 3 atomes d'halogènes; ou quand n est 0, alors $R_7$ peut aussi être un atome de chlore ou de brome, ou deux des $R_7$ quand ils sont situés sur des atomes de carbone adjacents forment ensemble un cycle époxyde ou une liaison carbone-carbone; ou quand n est 1, alors un $R_7$ sur l'atome de carbone adjacent un carbone portant $R_2$ est un groupe hydroxy, un groupe aralcoxy en $R_{7-11}$ ou un groupe alcoxy en $C_{1-4}$, et l'autre $R_7$ est un atome d'hydrogène;

les deux Q sont des atomes d'hydrogène ou des atomes de fluor; et

W représente un groupe cyano; un groupe alcényle ou alcynyle en $C_{2-4}$; un groupe pyrimidinyle, pyrazinyle, pyridazinyle, pyridyle, furyle, naphtyle, imidazolyle, triazolyle, thiadiazolyle, 2-quinolinyle, 1-isoquinolinyle, pyrrolyle, N-méthylimidazolyle, N-méthylpyrazolyle, isoxazolyle, oxazolyle, isothiazolyle, thiazolyle, thiényle ou 5-méthyl-2-furyle; ou un groupe cyclohexényle ou un groupe cycloalcoyle ayant jusqu'à 6 atomes de carbone éventuellement substitué par un groupe alcoyle en $C_{1-3}$; ou un groupe alcoyle secondaire en $C_{3-10}$; ou un groupe phényle qui est non-substitué ou substitué par une ou plusieurs des portions suivantes: hydroxy, cyano, nitro, halogène, alcoxy en $C_{1-3}$, haloalcoxy, alcoylthio, haloalcoylthio, alcoylsulfinyle, haloalcoylsulfinyle ou alcoylsulfonyle; alcényle ou alcynyle ayant jusqu'à 4 atomes de carbone; phényle, phénoxy, benzyle ou benzyloxy; amino, aminocarbonyle et carbonyle dans chacun desquels l'hydrogène peut être remplacé par un groupe alcoyle en $C_{1-4}$; et alcoyle en $C_{1-3}$ qui est lui-même éventuellement substitué par des substituants halogènes, hydroxy, $C_{1-4}$ alcoylthio, alcoxy en $C_{1-4}$ et amino.

2. Un composé selon la revendication 1, dans lequel chaque $R_4$ indépendamment représente un atome d'hydrogène ou un groupe méthyle ou éthyle.

3. Un composé selon la revendication 1 ou la revendication 2, dans lequel $R_5$ et $R_6$ représentent chacun indépendamment un atome d'hydrogène ou un groupe méthyle ou éthyle.

4. Un composé selon l'une quelconque des revendications 1 à 3, dans lequel chaque $R_7$ indépendamment représente un atome d'hydrogène ou un groupe méthyle ou éthyle ou, quand n est 0, un atome de chlore ou de brome ou, quand n est 1, un $R_7$ sur l'atome de carbone adjacent à l'atome de carbone portant $R_2$ est un groupe hydroxy, méthoxy, éthoxy ou benzyloxy, l'autre $R_7$ ou chaque autre $R_7$ étant un atome d'hydrogène.

5. Un composé selon la revendication 4, dans lequel chaque $R_7$ représente un atome d'hydrogène.

6. Un composé selon l'une quelconque des revendications 1 à 5, dans lequel n est 0 et p est 2.

7. Un composé selon l'une quelconque des revendications 1 à 5, dans lequel n est 1 et p est 2.

8. Un composé selon l'une quelconque des revendications 1 à 7, dans lequel W représente un groupe alcényle ou alcynyle en $C_{2-4}$; un groupe 4-pyramidinyle, 2-pyrazinyle, 3-pyridazinyle, 2-pyridyle ou 2-furyle; ou un groupé phényle éventuellement substitué par un ou plusieurs atomes d'halogène ou groupes cyano, amino, alcoxy en $C_{1-3}$ ou alcoylthio, chacun éventuellement substitué par un ou plusieurs atomes de fluor et/ou de chlore, ou alcoyle en $C_{1-2}$ éventuellement substitués par un ou plusieurs atomes de fluor et/ou de chlore ou par des groupes hydroxy, alcoxy en $C_{1-2}$ ou $C_{1-2}$ alcoylthio.

9. Un composé selon la revendication 8, dans lequel W représente un groupe éthynyle; un groupe 2-pyridyle; ou un groupe phényle qui est non-substitué ou substitué par un ou deux substituants choisi parmi des groupes méthyle, des atomes de fluor et des atomes de chlore.

10. Un composé selon l'une quelconque des revendications 1 à 9, dans lequel $R_1$ représente un groupe méthyle ou un atome d'hydrogène.

11. Un composé selon l'une quelconque des revendications 1 à 10, dans lequel $R_2$ représente un groupe méthyle ou éthyle.

12. Un composé selon l'une quelconque des revendications 1 à 11, dans lequel $R_3$ représente un atome d'hydrogène; un groupe alcoyle en $C_{1-6}$ éventuellement substitué par jusqu'à 3 atomes de fluor, de chlore et/ou de brome ou par un groupe hydroxy, cyano, alcoxy en $C_{1-6}$, $C_{1-6}$ alcoylsulfonyle, phénylsulfonyle ou benzylsulfonyle; un groupe alcényle ou alcynyle en $C_{2-4}$; ou un groupe aryle ou aralcoyle contenant 6 à 11 atomes de carbone et 1 ou 2 atomes de carbone dans toute portion alcoyle, éventuellement substitué au noyau par un ou plusieurs atomes de fluor, de chlore et/ou de brome ou par un groupe alcoyle ou alcoxy en $C_{1-2}$ éventuellement substitué par un ou plusieurs atomes de fluor et/ou de chlore.

13. Un composé selon la revendication 12, dans lequel $R_3$ représente un atome d'hydrogène ou un groupe alcoyle en $C_{1-3}$ éventuellement substitué par un ou plusieurs atomes d'halogènes.

14. Un composé selon l'une quelconque des revendications 1 à 13, dans lequel les deux Q représentent des atomes d'hydrogène.

15. Un composé selon la revendication 1, dans lequel $R_3$ est un atome d'hydrogène ou un groupe alcoyle en $C_{1-10}$ ou quand n est 0, alors $R_3$ peut être en outre un groupe cyano, un groupe alcoyle en $C_{1-6}$ substitué par un groupe hydroxy, un groupe cyano, un groupe $C_{1-6}$ alcoylsulfonyle, un groupe phénylsulfonyle, un groupe benzylsulfonyle, un groupe azido, un groupe ($C_{1-6}$ alcoxy)carbonyle, un groupe hydroxycarbonyle, un groupe phosphoryle, un groupe phosphoryloxy ou un groupe oxyde d'amine, carbamoyle ou thiocarbamoyle dans lequel chaque atome d'azote est éventuellement substitué par 1 ou 2 groupes alcoyle en $C_{1-4}$; ou $R_3$ est un groupe alcényle ou alcynyle en $C_{2-4}$; un groupe aryle ou aralcoyle, contenant chacun de 6 à 11 atomes de carbone comprenant 1 à 4 atomes de carbone dans toute portion alcoyle et éventuellement substitué au noyau par un ou plusieurs substituants choisi parmi des atomes de fluor, de chlore et/ou de brome, ou par un groupe alcoyle ou alcoxy en $C_{1-2}$, chacun éventuellement substitué par un ou plusieurs atomes de fluor et/ou de chlore; ou $R_3$ est un groupe $—CO_2R_8$ ou $—CON(R_8)_2$ où $R_8$ est un atome d'hydrogène ou un groupe alcoyle en $C_{1-6}$;

chaque $R_4$ est indépendamment un atome d'hydrogène ou un groupe alcoyle en $C_{1-6}$ éventuellement substitué par jusqu'à 3 atomes d'halogènes; ou un des $R_4$ et $R_1$ forment ensemble une liaison carbone-carbone; et

W est un groupe alcényle ou alcynyle contenant de 2 à 4 atomes de carbone; un groupe 4-pyrimidyle; un groupe 2-pyrazinyle; un groupe 3-pyridazinyle; un groupe 2-pyridyle; un groupe 2-furyle; ou un groupe phényle éventuellement substitué par un ou plusieurs atomes d'halogènes ou groupes cyano, amino, alcoxy en $C_{1-3}$ ou alcoylthio chacun éventuellement substitué par un ou plusieurs atomes de fluor et/ou de chlore, ou alcoyle en $C_{1-2}$ éventuellement substitué par un ou plusieurs atomes de fluor et/ou de chlore, hydroxy, alcoxy en $C_{1-2}$ ou $C_{1-2}$ alcoylthio.

16. Un procédé pour la préparation d'un composé tel que revendiqué dans la revendication 1, qui comprend la réaction d'un composé de la formule générale

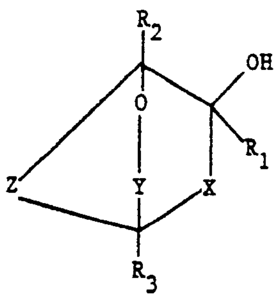

(II)

dans laquelle $R_1$, $R_2$, $R_3$, X, Y et Z ont les significations indiquées pour la formule générale I, avec un composé de la formule générale $WCQ_2L$, dans laquelle W et Q ont les significations indiquées pour la formule générale I et L représente un groupe qui part approprié.

17. Un procédé selon la revendication 16, mis en oeuvre en présence d'une base forte.

18. Un procédé selon la revendication 16 ou la revendication 17, mis en ouevure à une température comprise entre 0 et 120°C.

19. Un composé selon la revendication 1, chaque fois qu'il est préparé par un procédé selon l'une quelconque des revendications 16 à 18.

20. Une composition herbicide qui comprend un composé selon l'une quelconque des revendications 1 à 15 et 19 en même temps qu'un véhicule et/ou un agent tensio-actif.

21. Un procédé de lutte contre des plantes indésirables en un lieu, qui comprend le traitement du lieu par un composé selon l'une quelconque des revendications 1 à 15 et 19 ou par une composition selon la revendication 20.

**Revendications pour l'Etat contractant: AT**

1. Une composition herbicide qui comprend un ingrédient actif en même temps qu'un un véhicule et/ou un agent tensio-actif, caractérisée en ce que l'ingrédient actif est un composé de la formule générale I

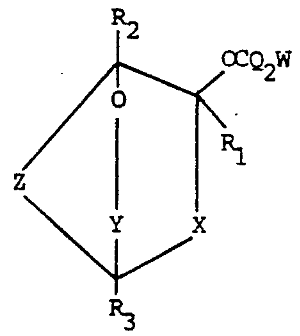

(I)

dans laquelle

X est $—CR_4R_4—$;

Y est $(—CR_5R_6—)_n$ où n est 0 ou 1;

Z est $(—CR_7R_7—)_p$ où p est 1, 2 ou 3;

$R_1$ est un atome d'hydrogène ou un groupe alcoyle en $C_{1-6}$ éventuellement substitué par jusqu'à 3 atomes de fluor, de chlore et/ou de brome;

$R_2$ est un atome d'hydrogène ou un groupe alcoyle en $C_{1-6}$ à chaîne droite;

$R_3$ est un atome d'hydrogène; un groupe cyano; un groupe alcoyle en $C_{1-10}$; un groupe alcoyle en $C_{1-6}$ qui est substitué par un ou plusieurs atomes d'halogènes ou par un groupe hydroxy, un groupe cyano, un groupe alcoxy en $C_{1-6}$, un groupe phénoxy, un groupe $C_{1-6}$ alcoylsulfonyle, un groupe phénylsulfonyle, un groupe benzylsulfonyle, un groupe azido, un groupe $C_{1-6}$ alcoxycarbonyle, un groupe benzyloxycarbonyle, un groupe hydroxycarbonyle, un groupe phosphoryle, un groupe phosphoryloxy ou un groupe oxyde d'amine, carbamoyle ou thiocarbamoyle dans lequel chaque atome d'azote est éventuellement substitué par 1 ou 2 groupes alcoyle en $C_{1-4}$; un groupe alcényle ou alcynyle en $C_{2-4}$; un groupe aryle ou aralcoyle, contenant chacun de 6 à 11 atomes de carbone comprenant 1 à 4 atomes de cabone dans toute portion alcoyle, éventuellement substitué au noyau par un ou plusieurs atomes de fluor, de chlore et/ou de brome ou par un groupe alcoyle ou alcoxy en $C_{1-2}$, chacun éventuellement substitué par un ou plusieurs atomes de fluor et/ou de chlore; un groupe $—CSNH_2$; un groupe $—CO_2R_8$ ou $—CON(R_8)_2$ où $R_8$ est un atome d'hydrogène ou un groupe alcoyle en $C_{1-6}$; ou un groupe acétyle ou un dérivé oxime ou acétal de ce groupe;

chaque $R_4$ est indépendamment un atome d'hydrogène; un groupe alcoyle en $C_{1-6}$ éventuellement substitué par jusqu'à 3 atomes d'halogènes; un groupe hydroxy; ou un groupe alcoxy en $C_{1-4}$; ou un des $R_4$ et $R_1$ forment ensemble une liaison carbone-carbone;

$R_5$ et $R_6$ sont chacun indépendamment un atome d'hydrogène ou un groupe alcoyle en $C_{1-2}$; ou quand ils sont situés sur un atome de carbone adjacent à l'atome d'oxygène du noyau, alors $R_5$ et $R_6$ peuvent former ensemble un groupe alcoylène contenant 4 ou 5 atomes de carbone;

chaque $R_7$ indépendamment est un atome d'hydrogène ou un groupe alcoyle en $C_{1-4}$ éventuellement substitué par jusqu'à 3 atomes d'halogènes; ou quand n est 0, alors $R_7$ peut aussi être un atome de chlore ou de brome, ou deux des $R_7$ quand ils sont situé sur des atomes de carbone adjacents forment ensemble un cycle époxyde ou une liaison carbone-carbone; ou quand n est 1, alors un $R_7$ sur l'atome de carbone adjacent un carbone portant $R_2$ est un groupe hydroxy, un groupe aralcoxy en $R_{7-11}$ ou un groupe alcoxy en $C_{1-4}$, et l'autre $R_7$ est un atome d'hydrogène;

les deux Q sont des atomes d'hydrogène ou des atomes de fluor; et

W représente un groupe cyano; un groupe alcényle ou alcynyle en $C_{2-4}$; un groupe pyrimidinyle, pyrazinyle, pyridazinyle, pyridyle, furyle, naphtyle, imidazolyle, triazolyle, thiadiazolyle, 2-quinolinyle, 1-isoquinolinyle, pyrrolyle, N-méthylimidazolyle, N-méthylpyrazolyle, isoxazolyle, oxazolyle, isothiazolyle, thiazolyle, thiényle ou 5-méthyl-2-furyle; ou un groupe cyclohexényle ou un groupe cycloalcoyle ayant jusqu'à 6 atomes de carbone éventuellement substitué par un groupe alcoyle en $C_{1-3}$; ou un groupe alcoyle secondaire en $C_{3-10}$; ou un groupe phényle qui est non-substitué ou substitué par une ou plusieurs des portions suivantes: hydroxy, cyano, nitro, halogène, alcoxy en $C_{1-3}$, halo-alcoxy, alcoylthio, halo-alcoylthio, alcoylsulfinyle, halo-alcoylsulfinyle ou alcoylsulfonyle; alcényle ou alcynyle ayant jusqu'à 4 atomes de carbone; phényle, phénoxy, benzyle ou benzyloxy; amino, aminocarbonyle et carbonyle dans chacun desquels l'hydrogène peut être remplacé par un groupe alcoyle en $C_{1-4}$; et alcoyle en $C_{1-3}$ qui est lui-même éventuellement substitué par des substituants halogènes, hydroxy, $C_{1-4}$ alcoylthio, alcoxy en $C_{1-4}$ et amino.

2. Une composition selon la revendication 1, dans laquelle chaque $R_4$ indépendamment représente un atome d'hydrogène ou un groupe méthyle ou éthyle.

3. Une composition selon la revendication 1 ou la revendication 2, dans lequel $R_5$ et $R_6$ représentent chacun indépendamment un atome d'hydrogène ou un groupe méthyle ou éthyle.

4. Une composition selon l'une quelconque des revendications 1 à 3, dans laquelle chaque $R_7$ indépendamment représente un atome d'hydrogène ou un groupe méthyle ou éthyle ou, quand n est 0, un atome de chlore ou de brome ou, quand n est 1, un $R_7$ sur l'atome de carbone adjacent à l'atome de carbone portant $R_2$ est un groupe hydroxy, méthoxy, éthoxy ou benzyloxy, l'autre $R_7$ ou chaque autre $R_7$ étant un atome d'hydrogène.

5. Une composition selon la revendication 4, dans laquelle chaque $R_7$ représente un atome d'hydrogène.

6. Une composition selon l'une quelconque des revendications 1 à 5, dans laquelle n est 0 et p est 2.

7. Une composition selon l'une quelconque des revendications 1 à 5, dans laquelle n est 1 et p est 2.

8. Une composition selon l'une quelconque des revendications 1 à 7, dans laquelle chaque W représente un groupe alcényle ou alcynyle en $C_{2-4}$; un groupe 4-pyrimidinyle, 2-pyrazinyle, 3-pyrazinyle, 2-pyridyle ou 2-furyle; ou un groupé phényle éventuellement substitué par un ou plusieurs atomes d'halogènes ou groupes cyano, amino, $C_{1-3}$ alcoxy ou alcoylthio, chacun éventuellement substitué par un ou plusieurs atomes de fluor et/ou de chlore, ou alcoyle en $C_{1-2}$ éventuellement substitué par un ou plusieurs atomes de fluor et/ou de chlore ou par des groupes hydroxy, alcoxy en $C_{1-2}$ ou $C_{1-2}$ alcoylthio.

9. Une composition selon la revendication 8, dans laquelle W représente un groupe éthynyle; un groupe 2-pyridyle; ou un groupe phényle qui est non-substitué ou substitué par un ou deux substituants choisi parmi des groupes méthyle, des atomes de fluor et des atomes de chlore.

10. Une composition selon l'une quelconque des revendications 1 à 9, dans laquelle $R_1$ représente un groupe méthyle ou un atome d'hydrogène.

11. Une composition selon l'une quelconque des revendications 1 à 10, dans laquelle $R_2$ représente un groupe méthyle ou éthyle.

12. Une composition selon l'une quelconque des revendications 1 à 11, dans laquelle $R_3$ représente un atome d'hydrogène; un groupe alcoyle en $C_{1-6}$ éventuellement substitué par jusqu'à 3 atomes de fluor, de chlore et/ou de brome ou par un groupe hydroxy, cyano, alcoxy en $C_{1-6}$, $C_{1-6}$ alcoylsulfonyle, phénylsulfonyle ou benzylsulfonyle; un groupe alcényle ou alcynyle en $C_{2-6}$; ou un groupe aryle ou aralcoyle contenant 6 à 11 atomes de carbone et 1 ou 2 atomes de carbone dans toute portion alcoyle, éventuellement substitué au noyau par un ou plusieurs atomes de fluor, de chlore et/ou de brome ou par un groupe alcoyle ou alcoxy en $C_{1-2}$ éventuellement substitué par un ou plusieurs atomes de fluor et/ou de chlore.

13. Une composition selon la revendication 12, dans laquelle $R_3$ représente un atome d'hydrogène ou un groupe alcoyle en $C_{1-3}$ éventuellement substitué par un ou plusieurs atomes d'halogènes.

14. Une composition selon l'une quelconque des revendications 1 à 13, dans laquelle les deux Q représentent des atomes d'hydrogène.

15. Une composition selon la revendication 1, dans laquelle $R_3$ est un atome d'hydrogène ou un groupe alcoyle en $C_{1-10}$ ou quand n est 0, alors $R_3$ peut être en outre un groupe cyano, un groupe alcoyle en $C_{1-6}$ substitué par un groupe hydroxy, un groupe cyano, un groupe $C_{1-6}$ alcoylsulfonyle, un groupe phénylsulfonyle, un groupe benzylsulfonyle, un groupe azido, un groupe ($C_{1-6}$ alcoxy)carbonyle, un groupe hydroxycarbonyle, un groupe phosphoryle, un groupe phosphoryloxy ou un groupe oxyde d'amine, carbamoyle ou thiocarbamoyle dans lequel chaque atome d'azote est éventuellement substitué par 1 ou 2 groupes alcoyle en $C_{1-4}$; ou $R_3$ est un groupe alcényle ou alcynyle en $C_{2-4}$; un groupe aryle ou aralcoyle, contenant chacun de 6 à 11 atomes de carbone comprenant 1 à 4 atomes de carbone dans toute portion alcoyle et éventuellement substitué au noyau par un ou plusieurs substituants choisi parmi des atomes de fluor, de chlore et/ou de brome, ou par un groupe alcoyle ou alcoxy en $C_{1-2}$, chacun éventuellement substitué par un ou plusieurs atomes de fluor et/ou de chlore; ou $R_3$ est un groupe $—CO_2R_8$ ou $—CON(R_8)_2$, où $R_8$ est un atome d'hydrogène ou un groupe alcoyle en $C_{1-6}$;

chaque $R_4$ est indépendamment un atome d'hydrogène ou un groupe alcoyle en $C_{1-6}$ éventuellement substitué par jusqu'à 3 atomes d'halogènes; ou un des $R_4$ et $R_1$ forment ensemble une liaison carbone-carbone; et

W est un groupe alcényle ou alcynyle contenant de 2 à 4 atomes de carbone; un groupe 4-pyrimidyle; un groupe 2-pyrazinyle; un groupe 3-pyridazinyle; un groupe 2-pyridyle; un groupe 2-furyle; ou un groupe phényle éventuellement substitué par un ou plusieurs atomes d'halogènes ou groupes cyano, amino, $C_{1-3}$ alcoxy ou alcoylthio, chacun éventuellement substitué par un ou plusieurs atomes de fluor et/ou de chlore, ou alcoyle en $C_{1-2}$ éventuellement substitué par un ou plusieurs atomes de fluor et/ou de chlore ou groupes hydroxy, alcoxy en $C_{1-2}$ ou $C_{1-2}$ alcoylthio.

16. Un procédé pour la préparation d'un composé tel que défini dans la revendication 1, qui comprend un composé de la formule générale

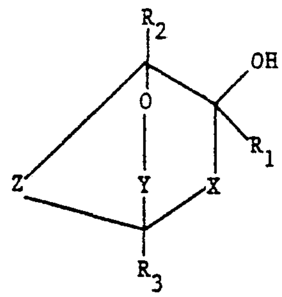

(II)

dans laquelle $R_1$, $R_2$, $R_3$, X, Y et Z ont les significations indiquées pour la formule générale I, avec un composé de la formule générale $WCQ_2L$, dans laquelle W et Q ont les significations indiquées pour la formule générale I, et L représente un groupe qui part approprié.

17. Un procédé selon la revendication 16, mis en oeuvre en présence d'une base forte.

18. Un procédé selon la revendication 16 ou la revendication 17, mis en ouevure à une température de 0 à 120°C.

19. Un procédé de lutte contre des plantes indésirables en un lieu, qui comprend le traitement du lieu par une composition selon une quelconque des revendications 1 à 15.